# EUROPEAN PATENT SPECIFICATION

(11) **EP 3 648 832 B1**
(45) Date of publication and mention of the grant of the patent: **24.11.2021**
(21) Application number: 18746059.7
(22) Date of filing: 03.07.2018
(51) Int. Cl.: A61N 1/02, A61N 1/372, A61N 1/05, A61N 1/06, A61N 1/36

(54) **SYSTEM FOR SELECTING STIMULATION CONFIGURATION AND TARGET FOR NEUROMODULATION**
SYSTEM ZUR AUSWAHL DER STIMULATIONSKONFIGURATION UND DES ZIELS FÜR DIE NEUROMODULATION
SYSTÈME SERVANT À SÉLECTIONNER UNE CONFIGURATION DE STIMULATION ET UNE CIBLE POUR LA NEUROMODULATION

(30) Priority: 06.07.2017 US 201762529167 P
(43) Date of publication of application: 13.05.2020
(73) Proprietor: Boston Scientific Neuromodulation Corporation, Valencia, CA 91355 (US)
(72) Inventor: ZHANG, Tianhe, Studio City California 91604 (US); ESTELLER, Rosana, Marietta Georgia 30068 (US)
(74) Representative: Vossius & Partner Patentanwälte Rechtsanwälte mbB
(86) International application number: PCT/US2018/040788
(87) International publication number: WO 2019/010225

(56) References cited:
- WO-A1-2007/097859
- US-A1- 2012 083 857
- US-A1- 2013 261 697
- US-A1- 2013 268 026
- US-B2- 7 933 656
- US-B2- 8 447 408
- Richard B North ET AL: "Spinal Cord Stimulator Adjustment to Maximize Implanted Battery Longevity: A Randomized, Controlled Trial Using a Computerized, Patient-Interactive Programmer", Neuromudulation: Technology at the Neural Interface, 1 January 2004 (2004-01-01), pages 13-25, XP055513446, Oxford, UK DOI: 10.1111/j.1525-1403.2004.04002.x Retrieved from the Internet: URL:https://onlinelibrary.wiley.com/doi/ab s/10.1111/j.1525-1403.2004.04002.x [retrieved on 2018-10-08]

## Description

### TECHNICAL FIELD

This document relates generally to medical devices and more particularly to a system and method for programing neuromodulation using relationship between stimulation configurations and neural targets.

### BACKGROUND

Neurostimulation, also referred to as neuromodulation, has been proposed as a therapy for a number of conditions. Examples of neurostimulation include Spinal Cord Stimulation (SCS), Deep Brain Stimulation (DBS), Peripheral Nerve Stimulation (PNS), and Functional Electrical Stimulation (FES). Implantable neurostimulation systems have been applied to deliver such a therapy. An implantable neurostimulation system may include an implantable neurostimulator, also referred to as an implantable pulse generator (IPG), and one or more implantable leads each including one or more electrodes. The implantable neurostimulator delivers neurostimulation energy through one or more electrodes placed on or near a target site in the nervous system. An external programming device is used to program the implantable neurostimulator with stimulation parameters controlling the delivery of the neurostimulation energy.

In one example, the neurostimulation energy is delivered in the form of electrical neurostimulation pulses. The delivery is controlled using stimulation parameters that specify spatial (where to stimulate), temporal (when to stimulate), and informational (patterns of pulses directing the nervous system to respond as desired) aspects of a pattern of neurostimulation pulses. Research has shown that the efficacy and efficiency of certain neurostimulation therapies can be improved, and their side-effects can be reduced, by delivering neurostimulation in a configuration tailored to each patient's specific needs and conditions. While modern electronics can accommodate the need for generating sophisticated neurostimulation configurations, the capability of a neurostimulation system depends on its post-manufacturing programmability to a great extent. For example, a stimulation configuration may only benefit a patient when it is customized for that patient, and stimulation configurations predetermined at the time of manufacturing may substantially limit the potential for the customization. Such customization may be performed at least in part by a user such as a physician or other caregiver with the patient in a clinical setting.

US 2012/083857 A1 relates to an external control device for use with a tissue stimulation device and at least one tissue stimulation lead having a plurality of electrodes implanted within a patient. The device comprises a user interface configured for allowing a user to enter first information defining a therapeutic indication and second information defining the location of the at least one tissue stimulation lead relative to an anatomical reference, at least one processor configured for analyzing the first and second information and generating a set of stimulation parameters based on the analysis, and output circuitry configured for transmitting the at least one stimulation parameter set to the tissue stimulation device.

US 2013/261697 A1 relates to devices for controlling spinal cord modulation for inhibiting pain, and associated systems and methods, including controllers for automated parameter selection are disclosed. A particular embodiment includes receiving a first input corresponding to a location of a signal delivery device implanted in a patient, establishing a positional relationship between the signal delivery device and an anatomical feature of the patient, receiving a second input corresponding to a medical indication of the patient, and, based at least in part on the positional relationship and the
indication, automatically identifying a signal delivery parameter in accordance with which a pulsed electrical signal is delivered to the patient via the signal delivery device.

WO 2007/097859 A1 relates to programming implantable stimulators to deliver stimulation energy via one or more implantable leads having complex electrode array geometries. The disclosure also contemplates guided programming to select electrode combinations and parameter values to support efficacy. The techniques may be applied to a programming interface associated with a clinician programmer, a patient programmer, or both. A user interface permits a user to view electrodes from different perspectives relative to the lead. For example, the user interface provides a side view of a lead and a, concentric axial view of the lead. The user interface may include an axial control medium to select and/or view electrodes at different axial positions along the length of a lead, and a rotational control medium to select and/or view electrodes at different angular positions around a circumference of the lead.

US 8,447,408 B2 relates to a control device to manipulate at least one neurostimulation parameter. A mapping system uses a calibrated map to map the directional output of the control device to values of at least one stimulation parameter to allow the user to intuitively control the value of the parameter. In some embodiments, where a stimulation device is used to deliver spinal cord stimulation (SCS) therapy for example, the user manipulates a parameter to effect the location and/or strength of paresthesia experienced by the patient. In exemplary embodiments, the parameter values are combinations of electrodes, and the mapping system selects electrode combinations based on the output of the control device such that a direction of movement of paresthesia experienced by the patient corresponds to a direction of manipulation of a directional controller of the control device. The mapping system may calibrate the map based on patient paresthesia information received from a user.

### SUMMARY

A system according to the invention is defined in claim 1. Any examples and embodiments referred to below which do not comprise all technical features of the system defined in claim 1 are not part of the invention.

An example (e.g., "Example 1") of a system for delivering neurostimulation to tissue of a patient using a stimulation device and a plurality of electrodes and controlling the delivery of the neurostimulation by a user is provided. The system may include a programming control circuit, a storage device, and a user interface. The programming control circuit may be configured to program the stimulation device for delivering the neurostimulation through one or more electrodes of the plurality of electrodes according to a stimulation configuration specified by a waveform parameter set defining a waveform of the neurostimulation and an electrode parameter set defining an electrode configuration of the neurostimulation. The storage device may be configured to store one or more neuromodulation relationships each relating one or more candidate stimulation configurations to one or more neural targets. The one or more candidate stimulation configurations are each selectable to be the stimulation configuration for programming the stimulation device. The one or more neural targets each specified by a target parameter set. The user interface may be coupled to the programming control circuit and the storage device, and may include modulation control circuitry. The modulation control circuitry may be configured to obtain two parameter sets selected from a group consisting of the waveform parameter set, the electrode parameter set, and the target parameter set, and configured to determine the other parameter set selected from the group consisting of the waveform parameter set, the electrode parameter set, and the target parameter set using the two obtained parameter sets and a neuromodulation relationship selected from the stored one or more neuromodulation relationships.

In Example 2, the subject matter of Example 1 may optionally be configured such that the modulation control circuitry is configured to obtain the waveform parameter set and the electrode parameter set, and to determine the target parameter set using the waveform parameter set, the electrode parameter set, and the selected neuromodulation relationship.

In Example 3, the subject matter of Example 1 may optionally be configured such that the modulation control circuitry is configured to obtain the waveform parameter set and the target parameter set, and determine the electrode parameter set using the waveform parameter set, the target parameter set, and the selected neuromodulation relationship.

In Example 4, the subject matter of Example 1 may optionally be configured such that the modulation control circuitry is configured to obtain the target parameter set and the electrode parameter set, and determine the waveform parameter set using the target parameter set, the electrode parameter set, and the selected neuromodulation relationship.

In Example 5, the subject matter of any one or any combination of Examples 1 to 4 may optionally be configured such that the modulation control circuitry includes relationship definition circuitry configured to obtain relationship information and configured to adjust a neuromodulation relationship selected from the stored one or more neuromodulation relationships using the obtained relationship information or to add a new neuromodulation relationship to the stored one or more neuromodulation relationships using the obtained relationship information.

In Example 6, the subject matter of Example 5 may optionally be configured such that the storage device is further configured to store one or more computational models, and the relationship definition circuitry is configured to adjust the selected neuromodulation relationship, or to add the new neuromodulation relationship, by performing a simulation using the obtained relationship information and one or more computation models selected from the stored one or more computational models.

In Example 7, the subject matter of any one or any combination of Examples 1 to 6 may optionally be configured such that the modulation control circuitry further includes stimulation definition circuitry and target definition circuitry. The stimulation definition circuitry is configured to obtain stimulation information and to produce one or more values for one or more parameters of the waveform parameter set and the electrode parameter set using the obtained stimulation information. The target definition circuitry configured to obtain target information and produce one or more values for one or more parameters of the target parameter set using the obtained target information.

In Example 8, the subject matter of Example 7 may optionally be configured such that the modulation control circuitry further includes stimulation configuration generation circuitry configured to generate the stimulation configuration for programming the stimulation device based on the produced one or more values for one or more parameters of the waveform parameter set, the electrode parameter set, and the target parameter set and a neuromodulation relationship selected from the stored one or more neuromodulation relationships.

In Example 9, the subject matter of Example 8 may optionally be configured such that the user interface includes a user input device, and the stimulation configuration generation circuitry is further configured to allow the user to select the neuromodulation relationship using the user input device.

In Example 10, the subject matter of any one or a combination of Examples 8 and 9 may optionally be configured such that the stimulation configuration generation circuitry is further configured to select the neuromodulation relationship selected automatically based on one or more of the obtained stimulation information or the obtained target information.

In Example 11, the subject matter of any one or any combination of Examples 8 to 10 may optionally be configured such that the user interface includes a presentation device, and the stimulation configuration generation circuitry is further configured to present on the presentation device the generated stimulation configuration as a recommendation for the stimulation configuration for programming the stimulation device.

In Example 12, the subject matter of Example 11 may optionally be configured such that the stimulation configuration generation circuitry is further configured to present a representation of the target parameter set corresponding to the recommended stimulation configuration.

In Example 13, the subject matter of any one or a combination of Examples 11 and 12 may optionally be configured such that the stimulation configuration generation circuitry is further configured to allow the user to adjust the generated stimulation configuration.

In Example 14, the subject matter of any one or any combination of Examples 7 to 13 may optionally be configured such that the storage device is further configured to store one or more disease-target relationships relating disease information indicative of one or more neural disorders to the neural targets, and the target definition circuitry is further configured to obtain the disease information as the target information and to produce the target parameters using the disease information and a disease-target relationship selected from the stored one or more disease-target relationships.

In Example 15, the subject matter of any one or any combination of Examples 1 to 14 may optionally be configured such that the modulation control circuitry further includes customization circuity configured to obtain customization information and customize one or more neuromodulation relationship selected from the stored one or more neuromodulation relationships using the customization information.

An example (e.g., "Example 16") of a method for controlling delivery of neurostimulation by a user is also provided. The neurostimulation is delivered to tissue of a patient from a stimulation device through a plurality of electrodes. The method may include storing one or more neuromodulation relationships each relating one or more candidate stimulation configurations to one or more neural targets. The one or more candidate stimulation configurations may each be specified by a waveform parameter set defining a waveform of the neurostimulation and an electrode parameter set defining an electrode configuration of the neurostimulation. The one or more neural target may each be specified by a target parameter set. The method may further include: obtaining two parameter sets of the waveform parameter set, the electrode parameter set, and the target parameter set; determining the other parameter set of the waveform parameter set, the electrode parameter set, and the target parameter set using the two obtained parameter sets and a neuromodulation relationship selected from the stored one or more neuromodulation relationships; selecting a stimulation configuration from the one or more candidate stimulation configurations based on the two obtained parameters sets and the determined other parameter set; and programming the stimulation device for delivering the neurostimulation through one or more electrodes of the plurality of electrodes according to the selected stimulation configuration.

In Example 17, the subject matter of obtaining the two parameter sets as found in Example 16 may optionally include obtaining the waveform parameter set and the electrode parameter set, and the subject matter of determining the other parameter set as found in Example 16 may optionally include determining the target parameter set.

In Example 18, the subject matter of obtaining the two parameter sets as found in Example 16 may optionally include obtaining the waveform parameter set and the target parameter set, and the subject matter of determining the other parameter set as found in Example 16 may optionally include determining the electrode parameter set.

In Example 19, the subject matter of obtaining the two parameter sets as found in Example 16 may optionally include obtaining the target parameter set and the electrode parameter set, and the subject matter of determining the other parameter set as found in Example 16 may optionally include determining the waveform parameter set.

In Example 20, the subject matter of any one or any combination of Examples 16 to 19 may optionally further include: determining the one or more neuromodulation relationships by performing a simulation using one or more computation models; storing the determined one or more neuromodulation relationships; and allowing for adjustment of each of the stored one or more neuromodulation relationships by the user.

In Example 21, the subject matter of storing the determined one or more neuromodulation relationships as found in Example 20 may optionally include storing the determined one or more neuromodulation relationships as look-up tables.

In Example 22, the subject matter of any one or a combination of Examples 20 and 21 may optionally further include: obtaining customization information specific to the patient; and customizing one or more neuromodulation relationship selected from the stored one or more neuromodulation relationships using the customization information.

In Example 23, the subject matter of any one or any combination of Examples 16 to 22 may optionally further include: obtaining stimulation information; producing one or more values for one or more parameters of the waveform parameter set and the electrode parameter set using the obtained stimulation information; obtaining target information; producing one or more values for one or more parameters of the target parameter set using the obtained target information; and generating the stimulation configuration for programming the stimulation device based on the produced one or more values for one or more parameters of the waveform parameter set, the electrode parameter set, and the target parameter set and a neuromodulation relationship selected from the stored one or more neuromodulation relationships.

In Example 24, the subject matter of any one or any combination of Examples 16 to 23 may optionally further include: presenting the selected stimulation configuration as a recommendation for the stimulation configuration for programming the stimulation device; and allowing the user to adjust the selected stimulation configuration using the user input device.

In Example 25, the subject matter of obtaining the target information as found in Example 23 may optionally include obtaining disease information, and the subject matter of producing the one or more values for the one or more parameters of the target parameter set as found in Example 23 may optionally include producing the one or more values using the disease information and a disease-target relationship relating the disease information to the neural targets.

This Summary is an overview of some of the teachings of the present application and not intended to be an exclusive or exhaustive treatment of the present subject matter. Further details about the present subject matter are found in the detailed description and appended claims. Other aspects of the disclosure will be apparent to persons skilled in the art upon reading and understanding the following detailed description and viewing the drawings that form a part thereof, each of which are not to be taken in a limiting sense. The scope of the present disclosure is defined by the appended claims and their legal equivalents.

### BRIEF DESCRIPTION OF THE DRAWINGS

Various embodiments are illustrated by way of example in the figures of the accompanying drawings. Such embodiments are demonstrative and not intended to be exhaustive or exclusive embodiments of the present subject matter.
FIG 1 illustrates a portion of a spinal cord.
FIG. 2 illustrates an embodiment of a neuromodulation system.
FIG. 3 illustrates an embodiment of a neuromodulation device, such as may be implemented in the neuromodulation system of FIG. 2.
FIG. 4 illustrates an embodiment of a programming device, such as may be implemented as the programming device in the neuromodulation system of FIG. 2.
FIG. 5 illustrates an implantable neuromodulation system and portions of an environment in which system may be used.
FIG. 6 illustrates an embodiment of a Spinal Cord Stimulation (SCS) system, which also may be referred to as a Spinal Cord Neuromodulation (SCM) system.
FIG. 7 illustrates an embodiment of neuromodulation leads and a pulse generator.
FIG. 8 illustrates an embodiment of a single electrical neuromodulation lead implanted over approximately the longitudinal midline of the patient's spinal cord.
FIG. 9 illustrates an embodiment where an electrical neuromodulation lead has been implanted more laterally with respect to the spinal cord, thereby placing it proximate the dorsal horn of the spinal cord, and the other electrical neuromodulation lead has been implanted more medially with respect to the spinal cord, thereby placing it proximate the dorsal column of the spinal cord.
FIG. 10 illustrates an embodiment of the electrical neuromodulation lead showing an example of the fractionalization of the anodic current delivered to the electrodes on the electrical neuromodulation lead.
FIGS. 11A-11B illustrate various embodiments of electrode arrangements and test regions of neural tissue along the electrode arrangements.
FIGS. 12A-12C illustrate examples of neural tissue locations that may be targeted within the test region in one, two and three dimensions, respectively.
FIG. 13 illustrates an embodiment of an implantable stimulator and one or more leads of an implantable neurostimulation system, such as the implantable neurostimulation system of FIG. 6.
FIG. 14 illustrates an embodiment of an external programming device of an implantable neurostimulation system according to the invention.
FIG. 15 illustrates an embodiment of a user interface of an external programming device, such as the external programming device of FIG. 14.
FIG. 16 illustrates an embodiment of a method for developing and using neuromodulation relationships relating stimulation configurations to neural targets.
FIG. 17 illustrates an embodiment of a look-up table (LUT) representing a neuromodulation relationship.
FIG. 18 illustrates an example of a display on a user interface, such as the user interface of FIG. 15, operating under a manual programming mode.
FIG. 19 illustrates another example of a display on a user interface, such as the user interface of FIG. 15, operating under the manual programming mode.
FIG. 20 illustrates an example of a display on a user interface, such as the user interface of FIG. 15, operating under a quick programming mode.
FIG. 21 illustrates another example of a display on a user interface, such as the user interface of FIG. 15, operating under the quick programming mode.
FIG. 22 illustrates an example of a display on a user interface, such as the user interface of FIG. 15, operating under a customization programming mode.
FIG. 23 illustrates another example of a display on a user interface, such as the user interface of FIG. 15, operating under the customization programming mode.
FIG. 24 illustrates an example of a display on a user interface, such as the user interface of FIG. 15, operating under the customization programming mode.

### DETAILED DESCRIPTION

The following detailed description of the present subject matter refers to the accompanying drawings which show, by way of illustration, specific aspects and embodiments in which the present subject matter may be practiced. These embodiments are described in sufficient detail to enable those skilled in the art to practice the present subject matter. Other embodiments may be utilized and structural, logical, and electrical changes may be made without departing from the scope of the present subject matter. References to "an", "one", or "various" embodiments in this disclosure are not necessarily to the same embodiment, and such references contemplate more than one embodiment. The following detailed description is, therefore, not to be taken in a limiting sense, and the scope is defined only by the appended claims.

This document relates to a neuromodulation system and method for configuring waveform and electrode patterns for selective stimulation of target tissue in a patient's nervous system. Neuromodulation relationships between various parameters, such as waveforms, electrode placements, spinal cord properties, and neural elements activated, can be developed using computational modeling and simulations and/or devices sensing signals from a patient and performing real-time computations. Such neuromodulation relationships relate neurostimulation parameters to various neural targets and/or disorders can be stored (e.g., as maps and/or look-up tables) for use in determining a stimulation configuration according to which neurostimulation is delivered. For example, a user may select a waveform and receive recommendations regarding neural targets, and/or may select one or more neural targets and receive recommendation for a waveform. After the user selects a recommended stimulation configuration and neural target(s), the parameters can be customized for each patient by accounting for patient-specific stimulation geometry and anatomical information that is acquired from the patient through surgery, imaging, and other anatomical studies. Examples of such patient-specific information can include depth of cerebrospinal fluid (CSF), medial lateral transverse diameter, distance between electrodes, and electrode-tissue coupling (e.g., tissue impedance and paresthesia threshold) for each electrode-tissue contact. In this document, a "user" includes any researcher, physician, other caregiver, or other trained professional who uses the present system and method to design, customize, and apply neuromodulation to a patient.

In various embodiments, the neuromodulation system can include a user interface that can operate in various modes to perform these functions. For example, the user interface can operate in a manual programming mode, a quick programming mode, and a customization programming mode. The manual programming mode allows an experienced user with expert knowledge to develop the neuromodulation relationships. The quick programming mode allows a field user to program neurostimulators for patients using stored neuromodulation relationships. The customization programming mode allows for adjustment of the recommended stimulation configuration using information specific to each patient.

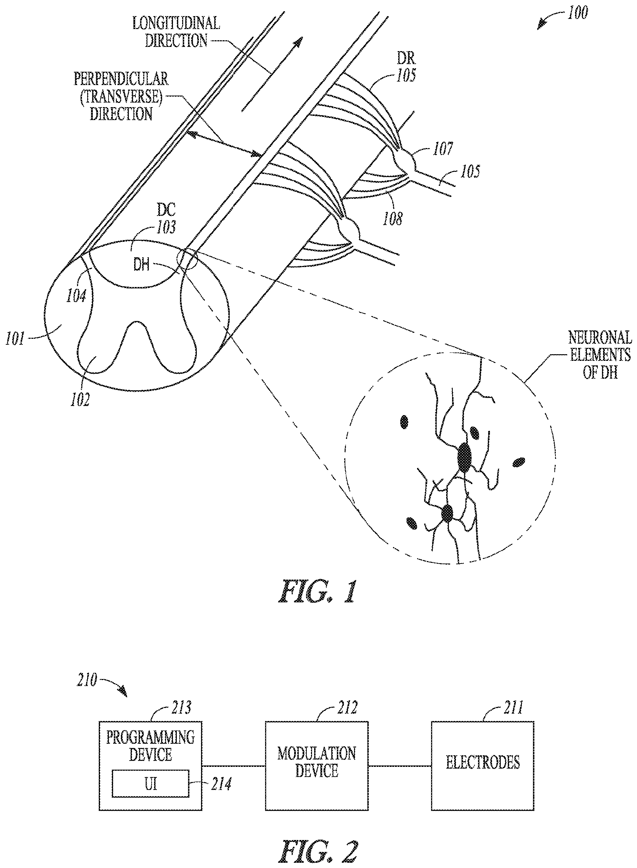

As some embodiments described herein involve Spinal Cord Stimulation (SCS, also referred to as spinal cord neuromodulation), a brief description of the physiology of the spinal cord is provided herein to assist the reader. FIG. 1 illustrates, by way of example, a portion of a spinal cord 100 including white matter 101 and gray matter 102 of the spinal cord. The gray matter 102 includes cell bodies, synapse, dendrites, and axon terminals. Thus, synapses are located in the gray matter. White matter 101 includes myelinated axons that connect gray matter areas. A typical transverse section of the spinal cord includes a central "butterfly" shaped central area of gray matter 102 substantially surrounded by an ellipse-shaped outer area of white matter 101. The white matter of the dorsal column (DC) 103 includes mostly large myelinated axons that form afferent fibers that run in an axial direction. The dorsal portions of the "butterfly" shaped central area of gray matter are referred to as dorsal horns (DH) 104. In contrast to the DC fibers that run in an axial direction, DH fibers can be oriented in many directions, including perpendicular to the longitudinal axis of the spinal cord. Examples of spinal nerves 105 are also illustrated, including a dorsal root (DR) 105, dorsal root ganglion 107 and ventral root 108. The dorsal root 105 mostly carries sensory signals into the spinal cord, and the ventral root functions as an efferent motor root. The dorsal and ventral roots join to form mixed spinal nerves 105.

SCS has been used to alleviate pain. A therapeutic goal for conventional SCS programming has been to maximize stimulation (i.e., recruitment) of the DC fibers that run in the white matter along the longitudinal axis of the spinal cord and minimal stimulation of other fibers that run perpendicular to the longitudinal axis of the spinal cord (dorsal root fibers, predominantly), as illustrated in FIG. 1. The white matter of the DC includes mostly large myelinated axons that form afferent fibers. While the full mechanisms of pain relief are not well understood, it is believed that the perception of pain signals is inhibited via the gate control theory of pain, which suggests that enhanced activity of innocuous touch or pressure afferents via electrical stimulation creates interneuronal activity within the DH of the spinal cord that releases inhibitory neurotransmitters (Gamma-Aminobutyric Acid (GABA), glycine), which in turn, reduces the hypersensitivity of wide dynamic range (WDR) sensory neurons to noxious afferent input of pain signals traveling from the dorsal root (DR) neural fibers that innervate the pain region of the patient, as well as treating general WDR ectopy. Consequently, the large sensory afferents of the DC nerve fibers have been targeted for stimulation at an amplitude that provides pain relief. Current implantable neuromodulation systems typically include electrodes implanted adjacent, *i.e.*, resting near, or upon the dura, to the dorsal column of the spinal cord of the patient and along a longitudinal axis of the spinal cord of the patient.

Activation of large sensory DC nerve fibers also typically creates the paresthesia sensation that often accompanies conventional SCS therapy. Although alternative or artifactual sensations, such as paresthesia, are usually tolerated relative to the sensation of pain, patients sometimes report these sensations to be uncomfortable, and therefore, they can be considered an adverse side-effect to neuromodulation therapy in some cases.

Some embodiments deliver sub-perception therapy that is therapeutically effective to treat pain, for example, but the patient does not sense the delivery of the neuromodulation field (e.g., paresthesia). Sub-perception therapy may be provided using neuromodulation of the spinal cord. Sub-perception neuromodulation may also be provided through neuromodulation field shaping (e.g., using multiple independent current control, or MICC), and temporal shaping of pulse train (e.g., burst, longer pulses). It appears that these higher frequencies may effectively block the transmission of pain signals in the afferent fibers in the DC. Some embodiments herein selectively modulate DH tissue or DR tissue over DC tissue to provide sub-perception therapy. Such selective neuromodulation may be delivered at lower frequencies. For example, the selective neuromodulation may be delivered at frequencies less than 1,200 Hz. The selective neuromodulation may be delivered at frequencies less than 1,000 Hz in some embodiments. In some embodiments, the selective neuromodulation may be delivered at frequencies less than 500 Hz. In some embodiments, the selective neuromodulation may be delivered at frequencies less than 350 Hz. In some embodiments, the selective neuromodulation may be delivered at frequencies less than 130 Hz. The selective neuromodulation may be delivered at low frequencies (e.g., as low as 2 Hz). The selective neuromodulation may be delivered even without pulses (i.e., DC stimulation) to modulate some neural tissue. By way of example and not limitation, the selective neuromodulation may be delivered within a frequency range selected from the following frequency ranges: 2 Hz to 1,200 Hz; 2 Hz to 1,000 Hz, 2 Hz to 500 Hz; 2 Hz to 350 Hz; or 2 Hz to 130 Hz. Systems may be developed to raise the lower end of any these ranges from 2 Hz to other frequencies such as, by way of example and not limitation, 10 Hz, 20 Hz, 50 Hz or 100 Hz. By way of example and not limitation, it is further noted that the selective neuromodulation may be delivered with a duty cycle, in which stimulation (e.g., a train of pulses) is delivered during a Stimulation ON portion of the duty cycle, and is not delivered during a Stimulation OFF portion of the duty cycle. By way of example and not limitation, the duty cycle may be about 10% ± 5%, 20% ± 5%, 30% ± 5%, 40% ± 5%, 50% ± 5% or 60% ± 5%. For example, a burst of pulses for 10 ms during a Stimulation ON portion followed by 15 ms without pulses corresponds to a 40% duty cycle.

While SCS is specifically discussed as an example of neuromodulation therapy, various embodiments can also include applying the priming techniques including timing of delivery discussed in this document to Peripheral Nerve Stimulation (PNS) therapies. For example, sub-perception PNS may be applied to alleviate pain. Various embodiments include priming the neural tissue at target locations for delivering the neuromodulation where required intensity of the neuromodulation for testing and/or therapeutic purposes may be lowered.

FIG. 2 illustrates an embodiment of a neuromodulation system. The illustrated system 210 includes electrodes 211, a neuromodulation device (also referred to as a "stimulation device") 212, and a programming device 213. The electrodes 211 are configured to be placed on or near one or more neural targets in a patient. The electrodes 211 may form part of an electrode arrangement. The neuromodulation device 212 is configured to be electrically connected to electrodes 211 and deliver neuromodulation energy, such as in the form of electrical pulses, to the one or more neural targets though electrodes 211. The delivery of the neuromodulation is controlled using a plurality of neuromodulation parameters, such as neuromodulation parameters specifying the electrical pulses and a selection of electrodes through which each of the electrical pulses is delivered. In various embodiments, at least some parameters of the plurality of neuromodulation parameters are programmable by a user, such as a physician or other caregiver. The programming device 213 provides the user with accessibility to the user-programmable parameters. In various embodiments, the programming device 213 is configured to be communicatively coupled to neuromodulation device via a wired or wireless link. In various embodiments, the programming device 213 includes a user interface (UI) 214 that allows the user to set and/or adjust values of the user-programmable neuromodulation parameters.

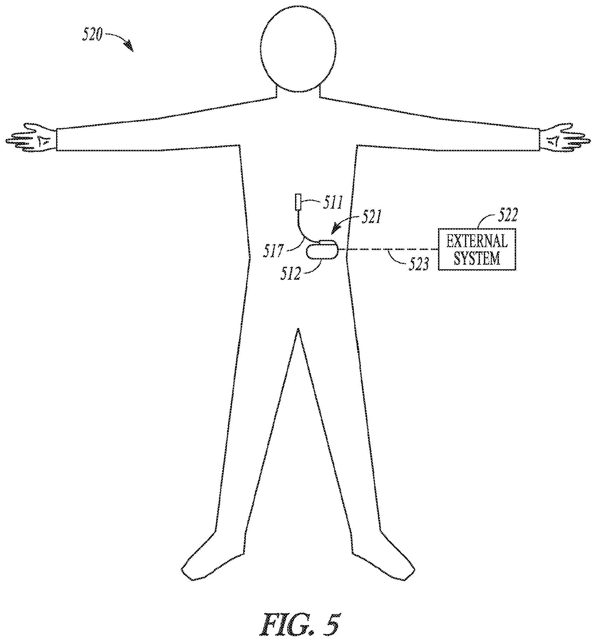

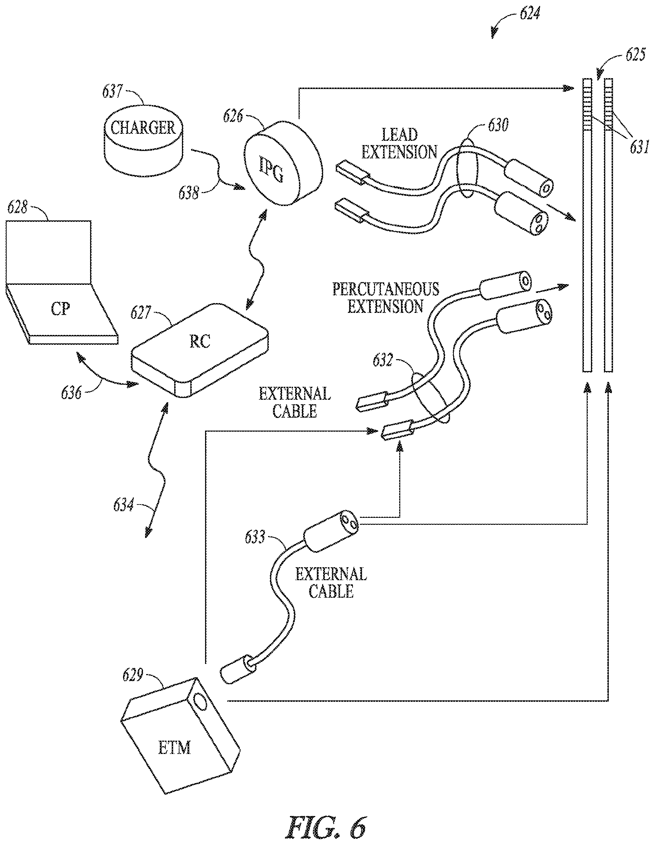

In various embodiments, the neuromodulation system 210 can include implantable and external elements. For example, the neuromodulation device 212 can be an implantable neuromodulation device, the electrodes 211 can include electrodes in one or more implantable lead and/or the implantable neuromodulation device, and the programming device can be an external programming device configured to be communicatively coupled to the implantable neuromodulation device via telemetry, as further discussed with reference to FIGS. 5 and 6. In another example, the neuromodulation device 212 can be an external neuromodulation device such as a Transcutaneous Electrical Neural Stimulation (TENS) device, the electrodes 211 can include surface electrodes such as skin patch electrodes, and the programming device can be an external programming device configured to be communicatively coupled to the implantable neuromodulation device via a wired or wireless link, or integrated with the external neuromodulation device. In still another example, the neuromodulation device 212 can be an external neuromodulation device, the electrodes 211 can include percutaneous electrodes, and the programming device can be an external programming device configured to be communicatively coupled to the implantable neuromodulation device via a wired or wireless link, or integrated with the external neuromodulation device. In various embodiments, an external neuromodulation device with surface and/or percutaneous electrodes can be used, for example, for delivering a test neuromodulation, delivering a therapeutic neuromodulation during a trial period, and delivering a short-term therapeutic neuromodulation.

In one embodiment, an external neuromodulation device with surface electrodes can be used during a trial period prior to a potential implantation of an implantable SCS system. A skin patch including the surface electrodes is placed over the patient's spine near the region where percutaneous electrodes will be placed for use during the trial period. The external neuromodulation device such as a dedicated External Trial Stimulator (ETC) and/or an external TENS device is used to prime the neural tissue before the trial period using one or more electrodes selected from the surface electrodes. This allows the programming of the external neuromodulation device for delivering therapeutic neuromodulation through the percutaneous electrodes to be performed with reduced wash-in time, such as immediately following the placement of the percutaneous electrodes.

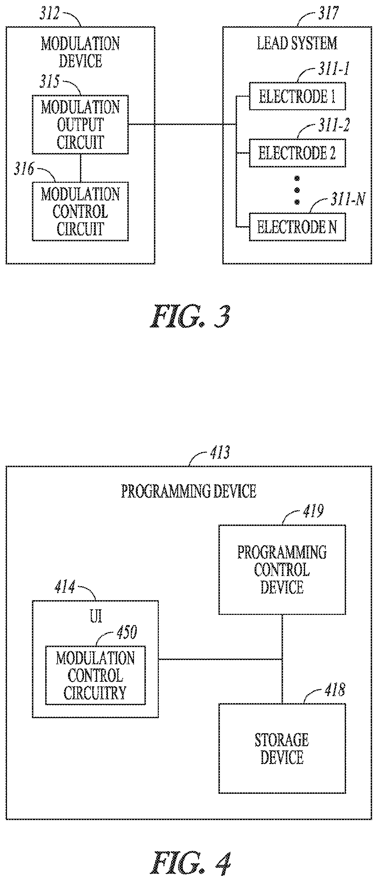

FIG. 3 illustrates an embodiment of a neuromodulation device 312, such as may be implemented in the neuromodulation system 210 of FIG. 2. The illustrated embodiment of the neuromodulation device 312 includes a neuromodulation output circuit 315 and a neuromodulation control circuit 316. Those of ordinary skill in the art will understand that the neuromodulation device 312 may include additional components such as sensing circuitry for patient monitoring and/or feedback control of the therapy, telemetry circuitry and power. The neuromodulation output circuit 315 produces and delivers neuromodulation pulses. The neuromodulation control circuit 316 controls the delivery of the neuromodulation pulses using the plurality of neuromodulation parameters. The combination of the neuromodulation output circuit 315 and neuromodulation control circuit 316 may collectively be referred to as a pulse generator. The lead system 317 includes one or more leads each configured to be electrically connected to neuromodulation device 3 12 and a plurality of electrodes 311-1 to 311 -N (where N ≥ 2) distributed in an electrode arrangement using the one or more leads. Each lead may have an electrode array consisting of two or more electrodes, which also may be referred to as contacts. Multiple leads may provide multiple electrode arrays to provide the electrode arrangement. Each electrode is a single electrically conductive contact providing for an electrical interface between neuromodulation output circuit 315 and tissue of the patient. The neuromodulation pulses are each delivered from the neuromodulation output circuit 315 through a set of electrodes selected from the electrodes 311-1 to 311-N. The number of leads and the number of electrodes on each lead may depend on, for example, the distribution of target(s) of the neuromodulation and the need for controlling the distribution of electric field at each target. In one embodiment, by way of example and not limitation, the lead system includes two leads each having eight electrodes.

The neuromodulation system may be configured to modulate spinal target tissue, brain tissue, or other neural tissue. The configuration of electrodes used to deliver electrical pulses to the targeted tissue constitutes an electrode configuration, with the electrodes capable of being selectively programmed to act as anodes (positive), cathodes (negative), or left off (zero). In other words, an electrode configuration represents the polarity being positive, negative, or zero. Other parameters that may be controlled or varied include the amplitude, pulse width, and rate (or frequency) of the electrical pulses. Each electrode configuration, along with the electrical pulse parameters, can be referred to as a "neuromodulation parameter set." Each set of neuromodulation parameters, including fractionalized current distribution to the electrodes (as percentage cathodic current, percentage anodic current, or off), may be stored and combined into a neuromodulation program that can then be used to modulate multiple regions within the patient.

The number of electrodes available combined with the ability to generate a variety of complex electrical pulses, presents a huge selection of neuromodulation parameter sets to the clinician or patient. For example, if the neuromodulation system to be programmed has sixteen electrodes, millions of neuromodulation parameter sets may be available for programming into the neuromodulation system. Furthermore, for example SCS systems may have thirty-two electrodes which exponentially increases the number of neuromodulation parameters sets available for programming. To facilitate such selection, the clinician generally programs the neuromodulation parameters sets through a computerized programming system to allow the optimum neuromodulation parameters to be determined based on patient feedback or other means and to subsequently program the desired neuromodulation parameter sets.

Conventional programming for SCS therapy uses paresthesia to select an appropriate neuromodulation parameter set. The paresthesia induced by the neuromodulation and perceived by the patient should be located in approximately the same place in the patient's body as the pain that is the target of treatment. When leads are implanted within the patient, an operating room (OR) mapping procedure may be performed to apply electrical neuromodulation to test placement of the leads and/or electrodes, thereby assuring that the leads and/or electrodes are implanted in effective locations within the patient. According to various embodiments, programming for sub-perception neuromodulation may prime the neural tissue to provide faster response times to the sub-perception neuromodulation as part of an OR mapping procedure.

Once the leads are correctly positioned, a fitting procedure, which may be referred to as a navigation session, may be performed to program the external control device, and if applicable the neuromodulation device, with a set of neuromodulation parameters that best addresses the painful site. Thus, the navigation session may be used to pinpoint the volume of activation (VOA) or areas correlating to the pain. The procedure may be implemented to target the tissue during implantation, or after implantation should the leads gradually or unexpectedly move that would otherwise relocate the neuromodulation energy away from the target site. By reprogramming the neuromodulation device (typically by independently varying the neuromodulation energy on the electrodes), the VOA can often be moved back to the effective pain site without having to re-operate on the patient in order to reposition the lead and its electrode array. According to various embodiments, a navigation session for sub-perception neuromodulation may prime the neural tissue to provide faster response times to the sub-perception neuromodulation.

Although various embodiments described in this document prime neural tissue to provide faster responses to sub-perception neuromodulation in order to perform faster OR mapping or navigation sessions, the present subject matter is not limited to such programming. By way of example and not limitation, some embodiment may prime the neural tissue before delivering the sub-perception neuromodulation therapy to the neural tissue simply to reduce the wash-in time of the therapy. Thus, by way of example, a patient may obtain pain relief much quicker with the primed neural tissue than without the primed neural tissue.

FIG. 4 illustrates an embodiment of a programming device 413, such as may be implemented as the programming device 213 in the neuromodulation system of FIG. 2. The programming device 413 includes a storage device 418, a programming control circuit 419, and a user interface (UI) 414. The programming control circuit 419 generates the plurality of neuromodulation parameters that controls the delivery of the neuromodulation pulses according to the pattern of the neuromodulation pulses. In various embodiments, user interface 414 includes any type of presentation device, such as interactive or non-interactive screens, and any type of user input devices that allow the user to program the neuromodulation parameters, such as touchscreen, keyboard, keypad, touchpad, trackball, joystick, and mouse. User interface 414 includes modulation control circuitry 450. In various embodiments, modulation control circuitry 450 can be configured to support one or more functions allowing for programming of neuromodulation devices, such as neuromodulation device 212, including its various embodiments as discussed in this document. The storage device 418 may store, among other things, neuromodulation parameters to be programmed into the neuromodulation device. The programming device 413 may transmit the plurality of neuromodulation parameters to the neuromodulation device. In some embodiments, the programming device 413 may transmit power to the neuromodulation device. The programming control circuit 419 may generate the plurality of neuromodulation parameters. In various embodiments, the programming control circuit 419 may check values of the plurality of neuromodulation parameters against safety rules to limit these values within constraints of the safety rules.

In various embodiments, circuits of neuromodulation, including its various embodiments discussed in this document, may be implemented using a combination of hardware, software and firmware. For example, the circuit of a UI, neuromodulation control circuit, and programming control circuit, including their various embodiments discussed in this document, may be implemented using an application-specific circuit constructed to perform one or more particular functions or a general-purpose circuit programmed to perform such function(s). Such a general-purpose circuit includes, but is not limited to, a microprocessor or a portion thereof, a microcontroller or portions thereof, and a programmable logic circuit or a portion thereof.

FIG. 5 illustrates, by way of example, an implantable neuromodulation system and portions of an environment in which system may be used. The system is illustrated for implantation near the spinal cord. However, neuromodulation system may be configured to modulate other neural targets such as may be useful for delivering other therapies. The system 520 includes an implantable system 521, an external system 522, and a telemetry link 523 providing for wireless communication between implantable system 521 and external system 522. The implantable system is illustrated as being implanted in the patient's body. The implantable system 521 includes an implantable neuromodulation device (also referred to as an implantable pulse generator, or IPG) 512, a lead system 517, and electrodes 511. The lead system 517 includes one or more leads each configured to be electrically connected to the neuromodulation device 512 and a plurality of electrodes 511 distributed in the one or more leads. In various embodiments, the external system 402 includes one or more external (non-implantable) devices each allowing a user (e.g., a clinician or other caregiver and/or the patient) to communicate with the implantable system 521. In some embodiments, the external system 522 includes a programming device intended for a clinician or other caregiver to initialize and adjust settings for the implantable system 521 and a remote control device intended for use by the patient. For example, the remote control device may allow the patient to turn a therapy on and off and/or adjust certain patient-programmable parameters of the plurality of neuromodulation parameters.

The neuromodulation lead(s) of the lead system 517 may be placed adjacent, i.e., resting near, or upon the dura, adjacent to the spinal cord area to be stimulated. For example, the neuromodulation lead(s) may be implanted along a longitudinal axis of the spinal cord of the patient. Due to the lack of space near the location where the neuromodulation lead(s) exit the spinal column, the implantable neuromodulation device 512 may be implanted in a surgically-made pocket either in the abdomen or above the buttocks, or may be implanted in other locations of the patient's body. The lead extension(s) may be used to facilitate the implantation of the implantable neuromodulation device 512 away from the exit point of the neuromodulation lead(s).

FIG. 6 illustrates, by way of example, an embodiment of a SCS system, which also may be referred to as a Spinal Cord Neuromodulation (SCM) system. The SCS system 624 may generally include a plurality (illustrated as two) of implantable neuromodulation leads 625, an implantable pulse generator (IPG) 626, an external remote controller RC 627, a clinician's programmer (CP) 628, and an external trial modulator (ETM) 629. The IPG 626 may be physically connected via one or more percutaneous lead extensions 630 to the neuromodulation leads 625, which carry a plurality of electrodes 631. As illustrated, the neuromodulation leads 625 may be percutaneous leads with the electrodes arranged in-line along the neuromodulation leads. Any suitable number of neuromodulation leads can be provided, including only one, as long as the number of electrodes is greater than two (including the IPG case function as a case electrode) to allow for lateral steering of the current. Alternatively, a surgical paddle lead can be used in place of one or more of the percutaneous leads. The IPG 626 includes pulse generation circuitry, also referred to as a pulse generator that delivers electrical neuromodulation energy in the form of a pulsed electrical waveform (*i.e*., a temporal series of electrical pulses) to the electrodes in accordance with a set of neuromodulation parameters.

The ETM 629 may also be physically connected via the percutaneous lead extensions 632 and external cable 633 to the neuromodulation leads 625. The ETM 629 may have similar pulse generation circuitry as the IPG 626 to deliver electrical neuromodulation energy to the electrodes accordance with a set of neuromodulation parameters. The ETM 629 is a non-implantable device that is used on a trial basis after the neuromodulation leads 625 have been implanted and prior to implantation of the IPG 626, to test the responsiveness of the neuromodulation that is to be provided. Functions described herein with respect to the IPG 626 can likewise be performed with respect to the ETM 629.

The RC 627 may be used to telemetrically control the ETM 629 via a bi-directional RF communications link 634. The RC 627 may be used to telemetrically control the IPG 626 via a bi-directional RF communications link 635. Such control allows the IPG 626 to be turned on or off and to be programmed with different neuromodulation parameter sets. The IPG 626 may also be operated to modify the programmed neuromodulation parameters to actively control the characteristics of the electrical neuromodulation energy output by the IPG 626. A clinician may use the CP 628 to program neuromodulation parameters into the IPG 626 and ETM 629 in the operating room and in follow-up sessions.

The CP 628 may indirectly communicate with the IPG 626 or ETM 629, through the RC 627, via an IR communications link 636 or other link. The CP 628 may directly communicate with the IPG 626 or ETM 629 via an RF communications link or other link (not shown). The clinician detailed neuromodulation parameters provided by the CP 628 may also be used to program the RC 627, so that the neuromodulation parameters can be subsequently modified by operation of the RC 627 in a stand-alone mode (i.e., without the assistance of the CP 628). Various devices may function as the CP 628. Such devices may include portable devices such as a lap-top personal computer, mini-computer, personal digital assistant (PDA), tablets, phones, or a remote control (RC) with expanded functionality. Thus, the programming methodologies can be performed by executing software instructions contained within the CP 628. Alternatively, such programming methodologies can be performed using firmware or hardware. In any event, the CP 628 may actively control the characteristics of the electrical neuromodulation generated by the IPG 626 to allow the desired parameters to be determined based on patient feedback or other feedback and for subsequently programming the IPG 626 with the desired neuromodulation parameters. To allow the user to perform these functions, the CP 628 may include a user input device (e.g., a mouse and a keyboard), and a programming display screen housed in a case. In addition to, or in lieu of, the mouse, other directional programming devices may be used, such as a trackball, touchpad, joystick, touch screens or directional keys included as part of the keys associated with the keyboard. An external device (e.g., CP) may be programmed to provide display screen(s) that allow the clinician to, among other functions, to select or enter patient profile information (e.g., name, birth date, patient identification, physician, diagnosis, and address), enter procedure information (e.g., programming/follow-up, implant trial system, implant IPG, implant IPG and lead(s), replace IPG, replace IPG and leads, replace or revise leads, explant, etc.), generate a pain map of the patient, define the configuration and orientation of the leads, initiate and control the electrical neuromodulation energy output by the neuromodulation leads, and select and program the IPG with neuromodulation parameters in both a surgical setting and a clinical setting.

An external charger 637 may be a portable device used to transcutaneously charge the IPG via a wireless link such as an inductive link 638. Once the IPG has been programmed, and its power source has been charged by the external charger or otherwise replenished, the IPG may function as programmed without the RC or CP being present.

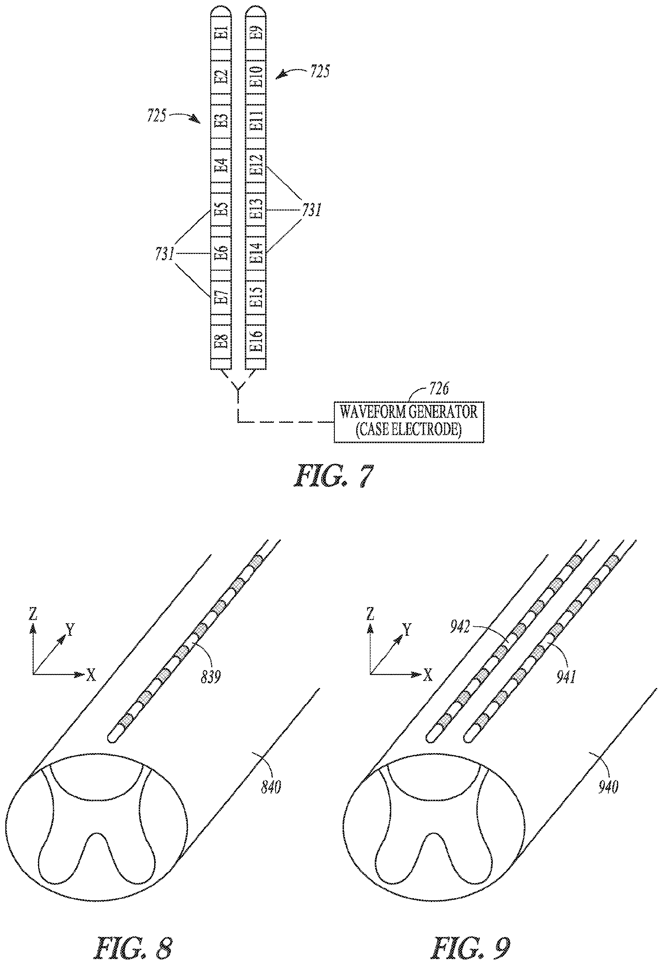

FIG. 7 illustrates, by way of example, some features of the neuromodulation leads 725 and a pulse generator 726. The pulse generator 726 may be an implantable device (IPG) or may be an external device such as may be used to test the electrodes during an implantation procedure. In the illustrated example, one of the neuromodulation leads has eight electrodes (labeled E1-E8), and the other neuromodulation lead has eight electrodes (labeled E9-E16). The actual number and shape of leads and electrodes may vary for the intended application. An implantable pulse generator (IPG) may include an outer case for housing the electronic and other components. The outer case may be composed of an electrically conductive, biocompatible material, such as titanium, that forms a hermetically-sealed compartment wherein the internal electronics are protected from the body tissue and fluids. In some cases, the outer case may serve as an electrode (e.g., case electrode). The IPG may include electronic components, such as a controller/processor (e.g., a microcontroller), memory, a battery, telemetry circuitry, monitoring circuitry, neuromodulation output circuitry, and other suitable components known to those skilled in the art. The microcontroller executes a suitable program stored in memory, for directing and controlling the neuromodulation performed by IPG.

Electrical neuromodulation energy is provided to the electrodes in accordance with a set of neuromodulation parameters programmed into the pulse generator. The electrical neuromodulation energy may be in the form of a pulsed electrical waveform. Such neuromodulation parameters may comprise electrode combinations, which define the electrodes that are activated as anodes (positive), cathodes (negative), and turned off (zero), percentage of neuromodulation energy assigned to each electrode (fractionalized electrode configurations), and electrical pulse parameters, which define the pulse amplitude (measured in milliamps or volts depending on whether the pulse generator supplies constant current or constant voltage to the electrode array), pulse width (measured in microseconds), pulse rate (measured in pulses per second), and burst rate (measured as the neuromodulation on duration X and neuromodulation off duration Y). The electrical pulse parameters may define an intermittent neuromodulation with "on" periods of time where a train of two or more pulses are delivered and "off" periods of time where pulses are not delivered. Electrodes that are selected to transmit or receive electrical energy are referred to herein as "activated," while electrodes that are not selected to transmit or receive electrical energy are referred to herein as "non-activated."

Electrical neuromodulation occurs between or among a plurality of activated electrodes, one of which may be the IPG case. The system may be capable of transmitting neuromodulation energy to the tissue in a monopolar or multipolar (e.g., bipolar, tripolar, etc.) fashion. Monopolar neuromodulation occurs when a selected one of the lead electrodes is activated along with the case of the IPG, so that neuromodulation energy is transmitted between the selected electrode and case.

Any of the electrodes E1-E16 and the case electrode may be assigned to up to k possible groups or timing "channels." In one embodiment, k may equal four. The timing channel identifies which electrodes are selected to synchronously source or sink current to create an electric field in the tissue to be stimulated. Amplitudes and polarities of electrodes on a channel may vary. In particular, the electrodes can be selected to be positive (anode, sourcing current), negative (cathode, sinking current), or off (no current) polarity in any of the k timing channels. The IPG may be operated in a mode to deliver electrical neuromodulation energy that is therapeutically effective and causes the patient to perceive delivery of the energy (e.g., therapeutically effective to relieve pain with perceived paresthesia, simulated natural-touch sensation, or other supraperception neuromodulation), and may be operated in a sub-perception mode to deliver electrical neuromodulation energy that is therapeutically effective and does not cause the patient to perceive delivery of the energy (e.g., therapeutically effective to relieve pain without perceived paresthesia). Some embodiments may use one channel to prime the neural tissue with a sub-perception neuromodulation field, and use another channel to deliver therapeutic sub-perception neuromodulation to the neural tissue.

The IPG may be configured to individually control the magnitude of electrical current flowing through each of the electrodes. For example, a current generator may be configured to selectively generate individual current-regulated amplitudes from independent current sources for each electrode. In some embodiments, the pulse generator may have voltage regulated outputs. While individually programmable electrode amplitudes are desirable to achieve fine control, a single output source switched across electrodes may also be used, although with less fine control in programming. Neuromodulators may be designed with mixed current and voltage regulated devices.

FIG. 8 is a schematic view of a single electrical neuromodulation lead 839 implanted over approximately the longitudinal midline of the patient's spinal cord 840. FIG. 9 illustrates an embodiment where an electrical neuromodulation lead 941 has been implanted more laterally with respect to the spinal cord, thereby placing it proximate the dorsal horn of the spinal cord, and the other electrical neuromodulation lead 942 has been implanted more medially with respect to the spinal cord, thereby placing it proximate the dorsal column of the spinal cord 940.

It is understood that additional leads or lead paddle(s) may be used, such as may be used to provide a wider electrode arrangement and/or to provide the electrodes closer to dorsal horn elements, and that these electrode arrays also may implement fractionalized current.

Placement of the lead more proximate to the DH than the DC may be desirable to preferentially stimulate DH elements over DC neural elements for a sub-perception therapy. Lead placement may also enable preferential neuromodulation of dorsal roots over other neural elements. Any other plurality of leads or a multiple column paddle lead can also be used. Longitudinal component of the electrical field is directed along the y-axis depicted in FIG. 8, and a transverse component of the electrical field is directed along the x-axis depicted in FIG. 8.

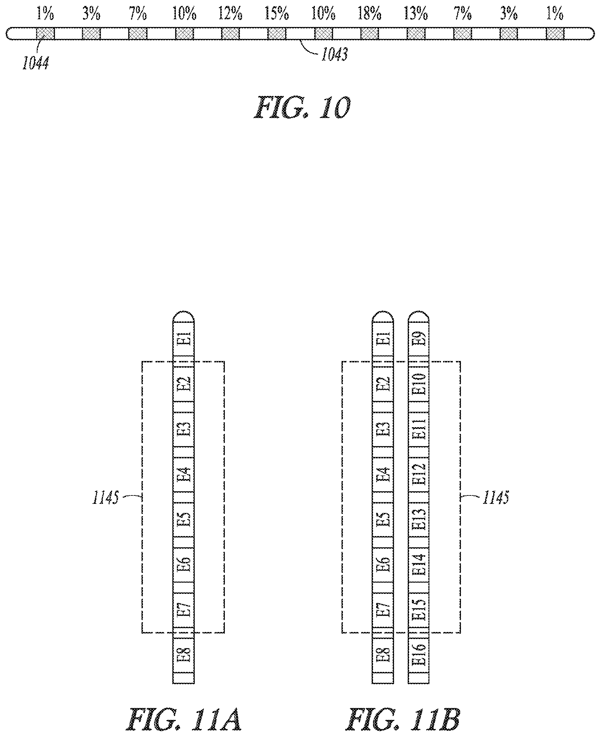

FIG. 10 is a schematic view of the electrical neuromodulation lead 1043 showing an example of the fractionalization of the anodic current delivered to the electrodes on the electrical neuromodulation lead. These figures illustrate fractionalization using monopolar neuromodulation where a case electrode of the IPG is the only cathode, and carries 100% of the cathodic current. The fractionalization of the anodic current shown in FIG. 10 does not deliver an equal amount of current to each electrode 1044, because this embodiment takes into account electrode / tissue coupling differences, which are the differences in how the tissue underlying each electrode reacts to electrical neuromodulation. Also, the ends of the portion of the electrical neuromodulation lead include electrodes having lower gradient in the longitudinal direction. The magnitude of the electrical field tapers down at the ends of the electrical neuromodulation lead. Fractionalization of the current may accommodate variation in the tissue underlying those electrodes. The fractionalization across the electrical neuromodulation lead can vary in any manner as long as the total of fractionalized currents equals 100%. Various embodiments described herein implement a programmed algorithm to determine the appropriate fractionalization to achieve a desired neuromodulation field property.

Neuromodulation thresholds vary from patient to patient and from electrode to electrode within a patient. An electrode / tissue coupling calibration of the electrodes may be performed to account for these different neuromodulation thresholds and provide a more accurate fractionalization of the current between electrodes. For example, perception threshold may be used to normalize the electrodes. The RC or the CP may be configured to prompt the patient to actuate a control element, once paresthesia is perceived by the patient. In response to this user input, the RC or the CP may be configured to respond to this user input by storing the neuromodulation signal strength of the electrical pulse train delivered when the control element is actuated. Other sensed parameter or patient-perceived neuromodulation values (e.g., constant paresthesia, or maximum tolerable paresthesia) may be used to provide the electrode / tissue coupling calibration of the electrodes.

The SCS system may be configured to deliver different electrical fields to achieve a temporal summation of neuromodulation. The electrical fields can be generated respectively on a pulse-by-pulse basis. For example, a first electrical field can be generated by the electrodes (using a first current fractionalization) during a first electrical pulse of the pulsed waveform, a second different electrical field can be generated by the electrodes (using a second different current fractionalization) during a second electrical pulse of the pulsed waveform, a third different electrical field can be generated by the electrodes (using a third different current fractionalization) during a third electrical pulse of the pulsed waveform, a fourth different electrical field can be generated by the electrodes (using a fourth different current fractionalized) during a fourth electrical pulse of the pulsed waveform, and so forth. These electrical fields may be rotated or cycled through multiple times under a timing scheme, where each field is implemented using a timing channel. The electrical fields may be generated at a continuous pulse rate, or may be bursted on and off. Furthermore, the interpulse interval (i.e., the time between adjacent pulses), pulse amplitude, and pulse duration during the electrical field cycles may be uniform or may vary within the electrical field cycle.

Some embodiments are configured to determine a neuromodulation parameter set to create a field shape to provide a broad and uniform neuromodulation field such as may be useful to prime targeted neural tissue with sub-perception neuromodulation. Some embodiments are configured to determine a neuromodulation parameter set to create a field shape to reduce or minimize neuromodulation of non-targeted tissue (e.g., DC tissue). The neuromodulation field may be shaped by using multiple independent current control (MICC) or multiple independent voltage control to guide the estimate of current fractionalization among multiple electrodes and estimate a total amplitude that provide a desired strength. For example, the neuromodulation field may be shaped to enhance the neuromodulation of DH neural tissue and to minimize the neuromodulation of DC tissue. A benefit of MICC is that MICC accounts for various in electrode-tissue coupling efficiency and perception threshold at each individual contact, so that "hot-spot" stimulation is eliminated.

Sub-perception SCS typically does not provide a quick feedback response regarding the effectiveness of the therapy. Rather, it has been observed that a wash-in period (a period of time for a delivered therapy to be therapeutically effective) for the sub-perception SCS is typically about one day. Thus, when the programmed neuromodulation parameters are changed to change the location of the sub-perception neuromodulation field, the patient may not be able to determine the effect that the changes have (e.g., pain relief) for a day or so. This make it difficult quickly titrate the neuromodulation field of the sub-perception SCS to provide effective pain relief to the patient.

It has been observed during research that priming the neural tissue enables faster pain relief feedback from the patient during the search for the neuromodulation field sweet spot. It may be appropriate to consider that priming the neural tissue "warms up" the neural tissue in a manner that reduces the wash-in time. However, neural physiology is complex and it is not currently understood why the primed neural tissue reduces the wash-in time of the sub-perception therapy such that the patient can quickly feel pain relief. It is noted that "priming" is different than conditioning pre-pulses which are delivered immediately before the neuromodulation pulse. A conditioning pre-pulse is timed to make a nerve more susceptible or less susceptible to capture by the immediately subsequent neuromodulation pulse. Thus, a conditioning pre-pulse has a specific relationship to a neuromodulation pulse. In contrast, the prime neuromodulation field extends over a much longer period of time. Further, rather than making neural tissue more or less excitable by a pulse, the prime neuromodulation field reduces a wash-in time of a therapy to make a patient feel the effects of the therapy (e.g., pain relief) much more quickly than would be felt without the prime field.

Various embodiments may deliver a low intensity, neuromodulation field in preparation to test for and find the sweet-spot for the neuromodulation field. The preparatory, lower intensity field is referred to herein as a prime field, as it is used to prime the neural tissue to be tested to have a quicker response to during the testing for the neuromodulation sweet spot for pain relief. The prime field can be a supra-perception or sub-perception neuromodulation field, but is typically even lower than the therapeutic sub-perception neuromodulation field.

A test region of neural tissue represents a region of tissue that is to be tested for a sweet spot. The test region may include many potential locations for targeting the neuromodulation field. The test region may span along the entire electrode arrangement (e.g., lead(s)) or may be reduced to a portion of the electrode arrangement. Priming may also be applied in a trolling fashion to cover the entire test region. As it is not known what location is to be most effective, the entire test region is primed.

In a non-limiting example to illustrate the lower intensity of the prime neuromodulation field, one may assume that a patient may feel paresthesia or otherwise perceive the delivery of the neuromodulation field when the neuromodulation current has an amplitude of 10 mA. Thus, 10 mA may be considered to be a perception threshold for the neuromodulation. Therapeutic sub-perception neuromodulation maybe delivered within a range of 30% to 90% of the perception threshold. Thus, in this example, neuromodulation with an amplitude between 3 mA and 9 mA may be therapeutically effective (e.g., provide pain relief). Priming the neural tissue may be accomplished using amplitudes near the lower range of the sub-perception neuromodulation or even below the lower range of the sub-perception neuromodulation such as, by way of example, between 2 mA to 4 mA. The sub-perception neuromodulation affects the neural tissue, but not to the point where the neuromodulation induces the nerve to trigger action potentials. Thus, the prime field may affect the ion concentrations within and outside of the neural pathways responsible for pain relief and/or may affect neurotransmitters responsible for pain relief, such that additional changes by sub-perception neuromodulation may more quickly induce desirable action potentials in these neural pathways responsible for pain relief.

FIGS. 11A-11B illustrate, by way of example and not limitation, electrode arrangements (e.g., E1-E8 in FIG. 11A and E1-E16 in FIG. 11B) and test regions 1145 of neural tissue along the electrode arrangements. These test regions 1145 may extend across the entire electrode arrangement. In some embodiments, the test regions may extend along only a portion of the electrode arrangement. By way of example, some embodiments may allow a user to select the test region and thus select the portion of the electrode arrangement to be tested. In the example illustrated in FIG. 11A the test region is neural tissue along the E2 to E7 electrodes, and in the example illustrated in FIG. 11B the test region is neural tissue along the E2 through E7 and the E10 to E15 electrodes.

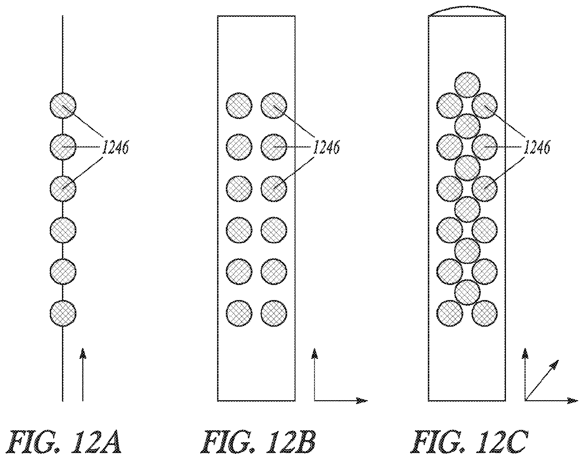

The electrodes in the electrode arrangement may be fractionalized, using different neuromodulation parameter sets, to change the portion of the neural tissue that is modulated. Thus, there may be many neural tissue locations that can be targeted with the test region of neural tissue adjacent to the electrode arrangement. FIGS. 12A-12C illustrate, by way of example and not limitation, neural tissue locations 1246 that may be targeted within the test region in one, two and three dimensions, respectively. In the one-dimensional example illustrated in FIG. 12A, the neural locations that may be targeted may simply be a line of potential targets such as may be observed from a single lead with a linear arrangement of electrodes. In the two dimensional example illustrated in FIG. 12B the neural locations that may be targeted may be considered to lie in a plane proximate to the electrode arrangement. In the three-dimensional example illustrated in FIG. 12C, the neural locations that may be targeted may be considered to be a volume of tissue proximate to the electrode arrangement. By way of example, the two-dimensional and three-dimensional test regions may be implemented using two or more leads of electrodes. Thus, the test regions may be relatively simple or complex shapes, and may include relatively few or relatively many locations to be tested.

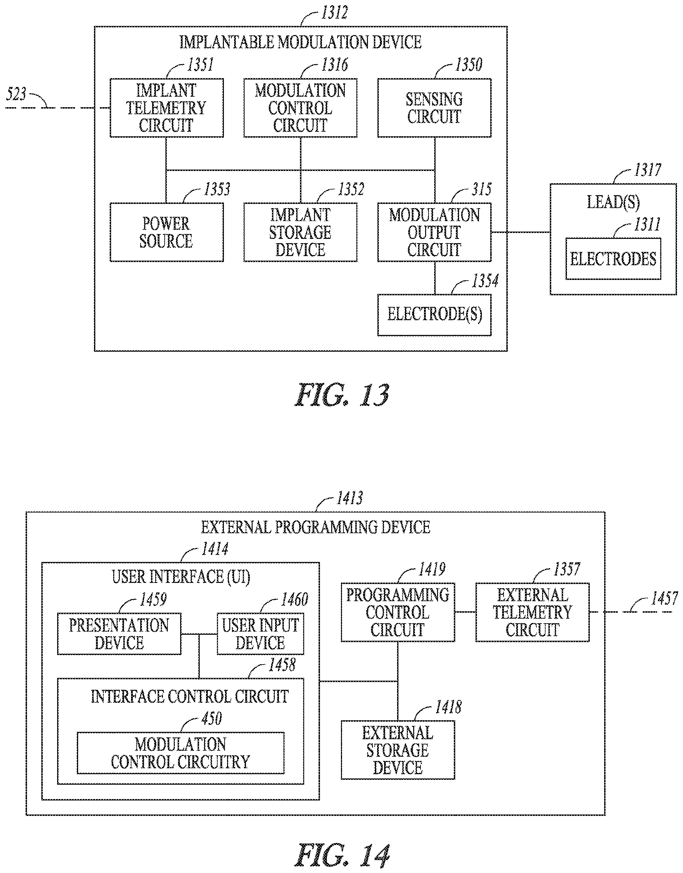

FIG. 13 illustrates an embodiment of implantable modulation device 1312 and one or more leads 1317 of an implantable neurostimulation system, such as implantable system 624. Implantable modulation device 1312 represents an embodiment of stimulation device 212 or 312 and may be implemented, for example, as IPG 604. Lead(s) 1317 represents an embodiment of lead system 317 and may be implemented, for example, as implantable leads 625. Lead(s) 1317 includes electrodes 1311, which represents an embodiment of electrodes 211 or 311 and may be implemented as electrodes 631.

Implantable modulation device 1312 may include a sensing circuit 1350 that is optional and required only when the stimulator needs a sensing capability, modulation output circuit 315, a modulation control circuit 1316, an implant storage device 1352, an implant telemetry circuit 1351, a power source 1353, and one or more electrodes 1354. Sensing circuit 1350, when included and needed, senses one or more physiological signals for purposes of patient monitoring and/or feedback control of the neurostimulation. Examples of the one or more physiological signals include neural and other signals each indicative of a condition of the patient that is treated by the neurostimulation and/or a response of the patient to the delivery of the neurostimulation. Modulation output circuit 315 is electrically connected to electrodes 706 through one or more leads 708 as well as electrodes 1354, and delivers each of the neurostimulation pulses through a set of electrodes selected from electrodes 706 and electrode(s) 1354. Modulation control circuit 1316 represents an embodiment of modulation control circuit 316 and controls the delivery of the neurostimulation pulses using the plurality of stimulation parameters specifying the pattern of neurostimulation pulses. In one embodiment, modulation control circuit 1316 controls the delivery of the neurostimulation pulses using the one or more sensed physiological signals. Implant telemetry circuit 1351 provides implantable modulation device 1312 with wireless communication with another device such as CP 630 and RC 632, including receiving values of the plurality of stimulation parameters from the other device. Implant storage device 1352 stores values of the plurality of stimulation parameters. Power source 1353 provides implantable modulation device 13 12 with energy for its operation. In one embodiment, power source 1353 includes a battery In one embodiment, power source 1353 includes a rechargeable battery and a battery charging circuit for charging the rechargeable battery. Implant telemetry circuit 1351 may also function as a power receiver that receives power transmitted from an external device through an inductive couple. Electrode(s) 1354 allow for delivery of the neurostimulation pulses in the monopolar mode. In various embodiments, electrode(s) 1354 (each of which may be referred to as a reference electrode) can be electrically connected to stimulation device 204, such as one or more electrodes each being a portion of or otherwise incorporated onto a housing of implantable modulation device 1312. Monopolar stimulation uses a monopolar electrode configuration with one or more electrodes selected from electrodes 311 and at least one electrode from electrode(s) 1354. Bipolar stimulation uses a bipolar electrode configuration with two electrodes selected from electrodes 311 and none electrode(s) 1354. Multipolar stimulation uses a multipolar electrode configuration with multiple (two or more) electrodes selected from electrodes 311 and none of electrode(s) 1354.

In one embodiment, implantable modulation device 1312 is used as a master database. A patient implanted with implantable modulation device 1312 (such as may be implemented as IPG 604) may therefore carry patient information needed for his or her medical care when such information is otherwise unavailable. Implant storage device 1352 is configured to store such patient information. For example, the patient may be given a new RC 632 and/or travel to a new clinic where a new CP 630 is used to communicate with the device implanted in him or her. The new RC 632 and/or CP 630 can communicate with implantable modulation device 1312 to retrieve the patient information stored in implant storage device 1352 through implant telemetry circuit 1351 and wireless communication link 523, and allow for any necessary adjustment of the operation of implantable modulation device 1312 based on the retrieved patient information. In various embodiments, the patient information to be stored in implant storage device 1352 may include, for example, positions of lead(s) 708 and electrodes 706 relative to the patient's anatomy (transformation for fusing computerized tomogram (CT) of post-operative lead placement to magnetic resonance imaging (MRI) of the brain), clinical effect map data, objective measurements using quantitative assessments of symptoms (for example using micro-electrode recording, accelerometers, and/or other sensors), and/or any other information considered important or useful for providing adequate care for the patient. In various embodiments, the patient information to be stored in implant storage device 1352 may include data transmitted to implantable modulation device 1312 for storage as part of the patient information and data acquired by implantable modulation device 1312, such as by using sensing circuit 1350.

In various embodiments, sensing circuit 1350 (if included), modulation output circuit 315, modulation control circuit 1316, implant telemetry circuit 1351, implant storage device 1352, and power source 1353 are encapsulated in a hermetically sealed implantable housing or case, and electrode(s) 707 are formed or otherwise incorporated onto the case. In various embodiments, lead(s) 708 are implanted such that electrodes 706 are placed on and/or around one or more targets to which the neurostimulation pulses are to be delivered, while implantable modulation device 1312 is subcutaneously implanted and connected to lead(s) 708 at the time of implantation.

FIG. 14 illustrates an embodiment of an external programming device 1413 of an implantable neurostimulation system, such as system 624. External programming device 1413 represents an embodiment of programming device 213 or 413, and may be implemented, for example, as CP 628 and/or RC 627. External programming device 1413 includes an external telemetry circuit 1451, an external storage device 1418, a programming control circuit 1419, and a user interface 1414.

External telemetry circuit 1451 provides external programming device 1413 with wireless communication with another device such as implantable modulation device 1312 via wireless communication link 523, including transmitting the plurality of stimulation parameters to implantable modulation device 1312 and receiving information including the patient data from implantable modulation device 1312. In one embodiment, external telemetry circuit 1451 also transmits power to implantable modulation device 1412 through an inductive couple.

In various embodiments, wireless communication link 523 can include an inductive telemetry link (near-field telemetry link) and/or a far-field telemetry link (RF telemetry link). For example, because DBS is often indicated for movement disorders which are assessed through patient activities, gait, balance, etc., allowing patient mobility during programming and assessment is useful. Therefore, when system 600 is intended for applications including DBS, wireless communication link 523 includes at least a far-field telemetry link that allows for communications between external programming device 1413 and implantable modulation device 1312 over a relative long distance, such as up to about 20 meters. External telemetry circuit 1451 and implant telemetry circuit 1451 each include an antenna and RF circuitry configured to support such wireless telemetry.

External storage device 1418 can store one or more stimulation waveforms for delivery during a neurostimulation therapy session, as well as various parameters and building blocks for defining one or more waveforms. The one or more stimulation waveforms may each be associated with one or more stimulation fields and represent a pattern of neurostimulation pulses to be delivered to the one or more stimulation field during the neurostimulation therapy session. In various embodiments, each of the one or more stimulation waveforms can be selected for modification by the user and/or for use in programming a stimulation device such as implantable modulation device 1312 to deliver a therapy. In various embodiments, each waveform in the one or more stimulation waveforms is definable on a pulse-by-pulse basis, and external storage device 1418 may include a pulse library that stores one or more individually definable pulse waveforms each defining a pulse type of one or more pulse types. External storage device 1418 also stores one or more individually definable stimulation fields. Each waveform in the one or more stimulation waveforms is associated with at least one field of the one or more individually definable stimulation fields. Each field of the one or more individually definable stimulation fields is defined by a set of electrodes through a neurostimulation pulse is delivered. In various embodiments, each field of the one or more individually definable fields is defined by the set of electrodes through which the neurostimulation pulse is delivered and a current distribution of the neurostimulation pulse over the set of electrodes. In one embodiment, the current distribution is defined by assigning a fraction of an overall pulse amplitude to each electrode of the set of electrodes. Such definition of the current distribution may be referred to as "fractionalization" in this document. In another embodiment, the current distribution is defined by assigning an amplitude value to each electrode of the set of electrodes. For example, the set of electrodes may include 2 electrodes used as the anode and an electrode as the cathode for delivering a neurostimulation pulse having a pulse amplitude of 4 mA. The current distribution over the 2 electrodes used as the anode needs to be defined. In one embodiment, a percentage of the pulse amplitude is assigned to each of the 2 electrodes, such as 75% assigned to electrode 1 and 25% to electrode 2. In another embodiment, an amplitude value is assigned to each of the 2 electrodes, such as 3 mA assigned to electrode 1 and 1 mA to electrode 2. Control of the current in terms of percentages allows precise and consistent distribution of the current between electrodes even as the pulse amplitude is adjusted. It is suited for thinking about the problem as steering a stimulation locus, and stimulation changes on multiple contacts simultaneously to move the locus while holding the stimulation amount constant. Control and displaying the total current through each electrode in terms of absolute values (e.g., mA) allows precise dosing of current through each specific electrode. It is suited for changing the current one contact at a time (and allows the user to do so) to shape the stimulation like a piece of clay (pushing/pulling one spot at a time).

External storage device 1418 can also store one or more neuromodulation relationships each relating one or more candidate stimulation configurations to one or more neural targets. The one or more candidate stimulation configurations are each a predetermined stimulation configuration that can be selected to be the stimulation configuration for programming a neuromodulation device such as neuromodulation device 212, including its various embodiments discussed in this document. In other words, if selected by the user, a candidate stimulation configuration becomes the stimulation configuration according to which the neuromodulation device is programmed. In various embodiments, external storage device 1418 can further store one or more computational models used for developing the one or more neuromodulation relationships, patient information including known information related to the stimulation configuration specific to the patient (e.g., the neuromodulation device and the plurality of electrodes used for, such as implanted in, the patient), and/or one or more disease-target relationships relating disease information (e.g., pain dermatome, pain etiology) to the neural targets. In various embodiments, the computational models stored in external storage device 1418 can include one or more estimators for neural activation. Such estimators include numeric values, such as the first order and second order spatial derivatives of the voltages in the space in the tissue, as well as any quantitative measures related to neural activity that do not require explicit simulation of neurons in a computational environment. Examples of such measure are discussed in Reilly, JP et al., "Sensory effects of transient electrical stimulation—evaluation with a neuroelectric model", IEEE Trans. Biomed. Eng., BME-32(12): 1001-11 (1985). In various embodiments, the one or more estimators of neural activation can be used in place of or in addition to other measures of neural activation, such as those derived from offline simulations.

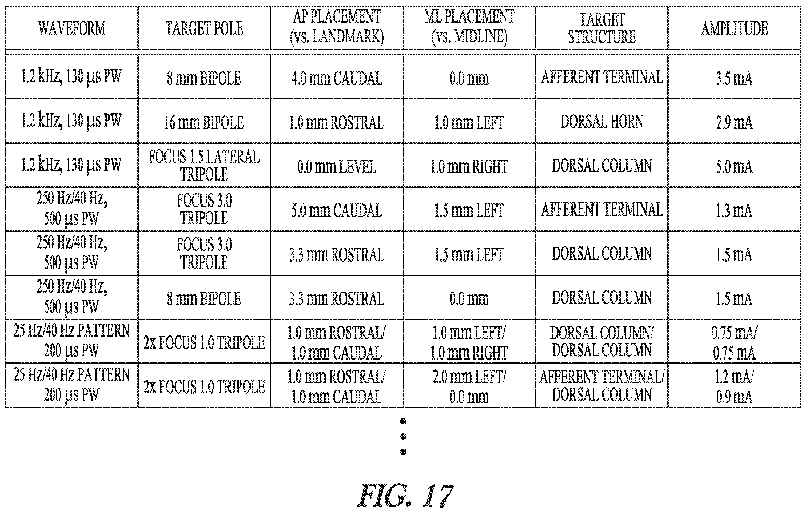

In various embodiments, the stimulation configuration can be specified by a waveform parameter set and an electrode parameter sets. For example, the stimulation configuration can define a pattern of neurostimulation pulses and a distribution of energy among the plurality of electrodes for delivering the neurostimulation pulses, where the distribution can vary as a function of time. The waveform parameter set can include one or more waveform parameters specifying the waveform of the neurostimulation. Examples of the waveform parameters include, but are not limited to, pulse frequency (stimulation rate, in Hz), pulse width (e.g., in µs), pulse amplitude (e.g., in mA), percentage cycling, modulation factor (for each of the pulse frequency, pulse width, pulse amplitude, and cycling), and waveform type (e.g., rectangular pulses, sinewave, triangular wave, exponential waveform, random waveform, and custom waveform). The electrode parameter set can include one or more electrode parameters specifying an electrode configuration used to deliver the neurostimulation. Examples of the electrode parameters include, but are not limited to, a lead design specifying location of each electrode of the plurality of electrodes relative to the locations of other electrodes of the plurality of electrodes, a lead placement indicative of anatomic location of each electrode when the lead is placed in the patient, an electrode selection indicative of active electrodes selected from the plurality of electrodes for delivering the neurostimulation, and a fractionalization indicative of a current flowing through each electrode of the plurality of electrodes. The lead placement can include anterior-posterior placement, which is relative to a specified reference point (structure), and/or medial-lateral placement, which is relative to the midline of the patient. The fractionalization can be specified as a percentage of the total current or specified as a current amplitude value (e.g., mA). The neural target can be specified by a target parameter set. The target parameter set can include one or more target parameters specifying the intended volume of activation by the neurostimulation. Examples of the target parameters include, but are not limited to, target poles and target structures (anatomic regions, e.g., dorsal horn, dorsal column, dorsal roots, root entry zone, rootlets, dorsal root ganglia, dorsal column nuclei, spinothalamic tract, Lissauer's tract, and other neural structures). In one embodiment, a neuromodulation relationship including the waveform parameter set, the electrode parameter set, and the target parameter set is established in the form of a look-up table (LUT), such as the LUT illustrated in FIG. 17 and discussed below.

In various embodiments, the one or more neuromodulation relationships each map each of one or more candidate stimulation configurations (each specified by a combination of parameter values for the waveform parameter set and the electrode parameter set) to a neural target of one or more neural targets (each specified by parameter values for the target parameter set). In various embodiments, the one or more neuromodulation relationships are each expressed in a form of a LUT including a plurality of candidate stimulation configurations mapped to a plurality of neural targets. In various embodiments, the one or more neuromodulation relationships are each established and/or adjusted for an individual patient. In various embodiments, the one or more neuromodulation relationships are each established and/or adjusted for a specified disorder. In various embodiments, the one or more neuromodulation relationships are each established and/or adjusted based on simulations using computational modeling.

Programming control circuit 1419 represents an embodiment of programming control circuit 316 and generates the plurality of stimulation parameters, which is to be transmitted to implantable modulation device 1312, based on a specified stimulation configuration (e.g., the pattern of neurostimulation pulses as represented by one or more stimulation waveforms and one or more stimulation fields, or at least certain aspects of the pattern). The stimulation configuration may be created and/or adjusted by the user using user interface 1414 and stored in external storage device 1418. In various embodiments, programming control circuit 1419 can check values of the plurality of stimulation parameters against safety rules to limit these values within constraints of the safety rules. In one embodiment, the safety rules are heuristic rules.

User interface 1414 represents an embodiment of user interface 310 and allows the user to define the pattern of neurostimulation pulses and perform various other monitoring and programming tasks. User interface 1414 includes a presentation device 1459, a user input device 1460, and an interface control circuit 1458. Presentation device 1459 may include any type of interactive or non-interactive display screens, and user input device 1460 may include any type of user input devices that supports the various functions discussed in this document, such as touchscreen, keyboard, keypad, touchpad, trackball, joystick, and mouse. In one embodiment, user interface 1414 includes a GUI. The GUI may also allow the user to perform any functions discussed in this document where graphical presentation and/or editing are suitable as may be appreciated by those skilled in the art.

Interface control circuit 1458 controls the operation of user interface 1414 including responding to various inputs received by user input device 1460 and defining the one or more stimulation waveforms. Interface control circuit 1458 includes modulation control circuitry 450.

In various embodiments, external programming device 1413 can have operation modes including a composition mode and a real-time programming mode. Under the composition mode (also known as the pulse pattern composition mode), user interface 1414 is activated, while programming control circuit 1419 is inactivated. Programming control circuit 1419 does not dynamically updates values of the plurality of stimulation parameters in response to any change in the one or more stimulation waveforms. Under the real-time programming mode, both user interface 1414 and programming control circuit 1419 are activated. Programming control circuit 1419 dynamically updates values of the plurality of stimulation parameters in response to changes in the set of one or more stimulation waveforms, and transmits the plurality of stimulation parameters with the updated values to implantable modulation device 1312.

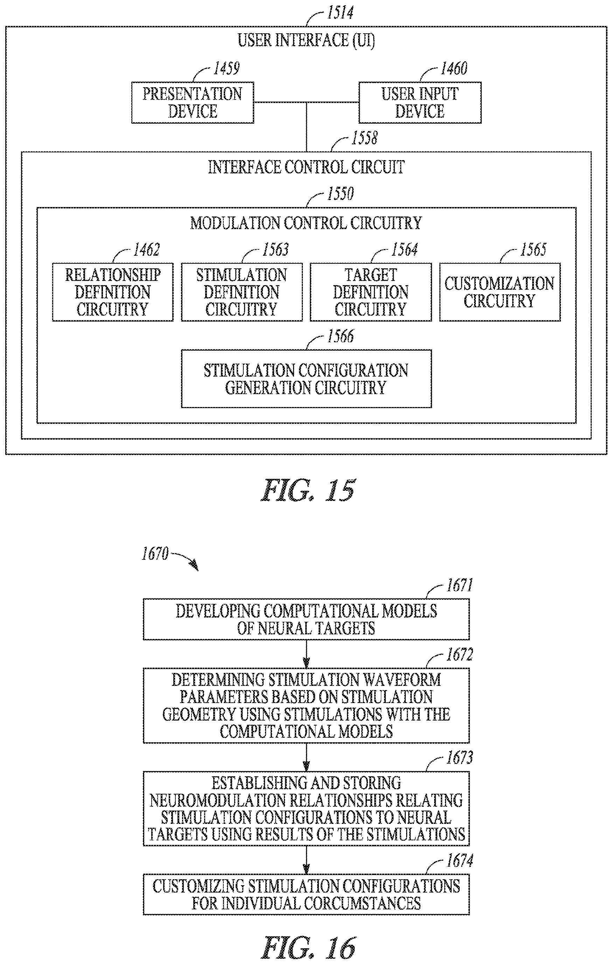

FIG. 15 illustrates an embodiment of a user interface 1514 of an external programming device, such as external programming device 1413. User interface 1514 represents an example of user interface 1414 and allows the user to define the stimulation configuration and perform various other monitoring and programming tasks. User interface 1514 includes presentation device 1459, user input device 1460, and an interface control circuit 1550. Presentation device 1359 may include any type of interactive or non-interactive screens, and user input device 1360 may include any type of user input devices that supports the various functions discussed in this document, such as touchscreen, keyboard, keypad, touchpad, trackball, joystick, and mouse. In one embodiment, user interface 1514 includes a graphical user interface (GUI) that allows the user to perform any functions discussed in this document where graphical presentation and/or editing are suitable as may be appreciated by those skilled in the art.

Interface control circuit 1558 represents an embodiment of interface control circuit 1458 and includes a modulation control circuitry 1550, which represents an embodiment of modulation control circuitry 450 and determines the stimulation configuration for programming a neuromodulation device, such as implantable modulation device 1312 or any other embodiment of neuromodulation device 212. Modulation control circuitry 1550 can include relationship definition circuitry 1562, stimulation definition circuitry 1563, target definition circuitry 1564, customization circuity 1565, and stimulation configuration generation circuitry 1566.

Relationship definition circuitry 1562 can obtain relationship information and adjust a neuromodulation relationship selected from the one or more neuromodulation relationships stored in external storage device 1418 using the obtained relationship information, or add a new neuromodulation relationship to the stored one or more neuromodulation relationships using the obtained relationship information. In one embodiment, relationship definition circuitry 1562 adjusts or creates the neuromodulation relationship by performing a simulation using the obtained relationship information and one or more computation models selected from the one or more computational models stored in external storage device 1418.

Stimulation definition circuitry 1563 can obtain stimulation information and produce one or more values for one or more parameters of the waveform parameter set and/or one or more values for one or more parameters of the electrode parameter set using the obtained stimulation information. Target definition circuitry 1564 can obtain target information and produce one or more values for one or more parameters of the target parameter set using the obtained target information.

Customization circuity 1565 can obtain customization information and customize one or more neuromodulation relationship selected from one or more neuromodulation relationships stored in external storage device 1418 using the customization information. Examples of the customization information include, but are not limited to patient-specific information such as depth of cerebrospinal fluid (CSF), medial lateral transverse diameter, distance between electrodes, and electrode-tissue coupling (e.g., tissue impedance and paresthesia threshold) for each electrode-tissue contact).

Stimulation configuration generation circuitry 1566 can generate the stimulation configuration for programming the neuromodulation device based on the one or more values for one or more parameters of the waveform parameter set, the electrode parameter set, and the target parameter set, produced by stimulation definition circuitry 1563 and/or target definition circuitry 1564, and a neuromodulation relationship selected from one or more neuromodulation relationships stored in external storage device 1418. The neuromodulation relationship can be selected by the user using user input device 1460 or selected automatically by stimulation configuration generation circuitry 1566 based on the obtained target information and/or customization information. In various embodiments, stimulation configuration generation circuitry 1566 can present on presentation device 1459 the generated stimulation configuration as a recommendation for the stimulation configuration for programming the neuromodulation device, and can also present on presentation device 1459 a representation of the target parameter set corresponding to the recommended stimulation configuration. Stimulation configuration generation circuitry 1566 can then allow the user to adjust the generated stimulation configuration and/or the target parameter set. Such adjustment can be performed by entering additional stimulation information and/or additional target information for generating a new stimulation configuration and/or modifying the stimulation configuration directly. The adjustment can be a reiterative process performed until the user accepts the stimulation configuration for programming the neuromodulation device.

In various embodiments, after one or more neuromodulation relationships are developed and stored, modulation control circuitry 1550 can use a neuromodulation relationship selected from the stored one or more neuromodulation relationships and known parameters of the stimulation configuration to determine the unknown parameters of the stimulation configuration (which is specified by the waveform parameter set, the electrode parameter set, and the target parameter set). For example, modulation control circuitry 1550 can obtain two parameter sets of the waveform parameter set, the electrode parameter set, and the target parameter set, and determine the other parameter set of the waveform parameter set, the electrode parameter set, and the target parameter set using the two obtained parameter sets and the selected neuromodulation relationship. Thus, depending on the information available, modulation control circuitry 1550 can (1) obtain the waveform parameter set and the electrode parameter set, and determine the target parameter set, (2) obtain the waveform parameter set and the target parameter set, and determine the electrode parameter set, and (3) obtain the target parameter set and the electrode parameter set, and determine the waveform parameter set.

In various embodiments, modulation control circuitry 1550 including each of its components can "obtain" various information by, for example, receiving from the user though user input device 1460, selecting from information stored in external storage device 1418, obtaining from elsewhere in the system such as system 210, system 520, and system 624, and/or communicated from another system. Thus, "obtained" information can include information obtained from any person or device by any manual and/or automatic means.

FIG. 16 illustrates an embodiment of a method 1670 for developing and using neuromodulation relationships relating the candidate stimulation configurations to the neural targets. Which neural elements are activated depends on the stimulation configuration, including both the waveform and the electrode configuration. For example, placement (e.g., rostral vs. caudal placement) of an electrode may determine which neural elements are activated at lowest stimulation intensities. The neuromodulation relationship may vary across waveforms (e.g., less-than 10 kHz pulse frequency and greater-than 70 µs pulse width vs. 10 kHz pulse frequency and 30 µs pulse width). Depending on the order of recruitment assessed from activation threshold specific to the patient, a type of the waveform and specific values or ranges of values of waveform parameters can be recommended to the user using the neuromodulation relationship. In one embodiment, system 210, including its various embodiments discussed in this document, can be used by the user to perform method 1670.

At 1671, computational models of neural targets (neural elements that can potentially be targeted for delivering neurostimulation) are developed. In one embodiment, computational models including finite element models (FEMs) are developed according to realistic anatomic and physiological parameters from academic literature. Computer-aided-design (CAD) software can be used to render the geometry of the spinal cord and then import that to an FEM solver. Simulations of neuron activity are conducted by calculating potentials, currents, and other electrical effects of stimulation alone using the FEM, extracting those values from the FEM solver, and coupling those values to a biophysical mathematical model in another software platform. In various embodiments, when available, dimensions of then patient's spinal cord and stimulation geometry (i.e., anatomy, target pole, and electrode geometry) taken from patient imaging data and/or known anatomical atlas are used in developing the computational models. When such data are not available, the computational models can be developed based on the patient's gender, height, weight, age, electrode impedance and/or other population specific parameters allowing for estimation of spinal cord dimensions and stimulation geometry. In some embodiments, the computational models can include one or more estimators for neural activation, such as the first order and second order spatial derivatives of electric potential in space in tissue and/or other one or more quantitative measures related to neural activity as discussed above with reference to FIG. 14.

At 1672, stimulation waveform parameters are determined based on stimulation geometry using simulations with the computational models. For a given stimulation geometry, voltages, electric fields, currents, etc., are calculated using the computational models. Calculated results can be varied in time by scaling with an arbitrary stimulation waveform. Neural activation/modulation produced by neurostimulation can then be simulated using a computational biophysical simulator (e.g., NEURON, MATLAB, or Brian2), and both the lowest threshold of modulation (e.g., determined using binary search for excitatory or inhibitory threshold) and the first active element at which signs of neural activation or modulation occur (e.g., by action potential counts and timing of effect) can be determined for a given anatomy, waveform, and electrode configuration.

At 1673, the neuromodulation relationships relating stimulation configurations to neural targets are established using results of the simulations. For a given stimulation geometry, the first active element, amplitude at which activation or modulation (e.g., inhibition via detection of a reduction in action potential counts) occurs, and the stimulation waveform are linked and stored in a storage medium with configurable memory. This storage medium can then be used to drive a user interface that allows a user to retrieve a recommended waveform after selecting a neural target and electrode configuration, a neural target after specifying an electrode geometry and waveform, an electrode geometry after specifying a waveform and neural target, or any combination thereof.

At 1674, stimulation configurations are customized for individual circumstances. Different disease states may have different neural substrates, so the user can select a disease state or official diagnosis as a proxy for the neural target. The user can customize and/or change settings across patients by determining inputs to the finite element model for an on-board simulation or inverse calculation platform through imaging data and/or clinical observations. The user can also receive different recommendations based on the neural target(s) underlying a disease state and/or other diagnostic data that the user can specify. The user can also request optimal parameters or optimal range of parameters for specific waveform or electrode configuration (e.g., parameters that yield optimal paresthesia and pain overlap). The user can also introduce specific target dermatomes to the system so the system can provide the stimulation configuration.

FIG. 17 illustrates an embodiment of a look-up table (LUT) representing a neuromodulation relationship such as one of the one or more neuromodulation relationships stored in external storage device 1418 and used by modulation control circuitry 1550. The illustrated LUT includes relationship between the waveform, electrode, and target parameters that may be generated off-line in a common database or in a patient-specific manner with an on-board application (e.g., installed in CP 628) based on clinical observations. The LUT may be developed or adjusted based on disease state, as different disease states may be simulated separately.

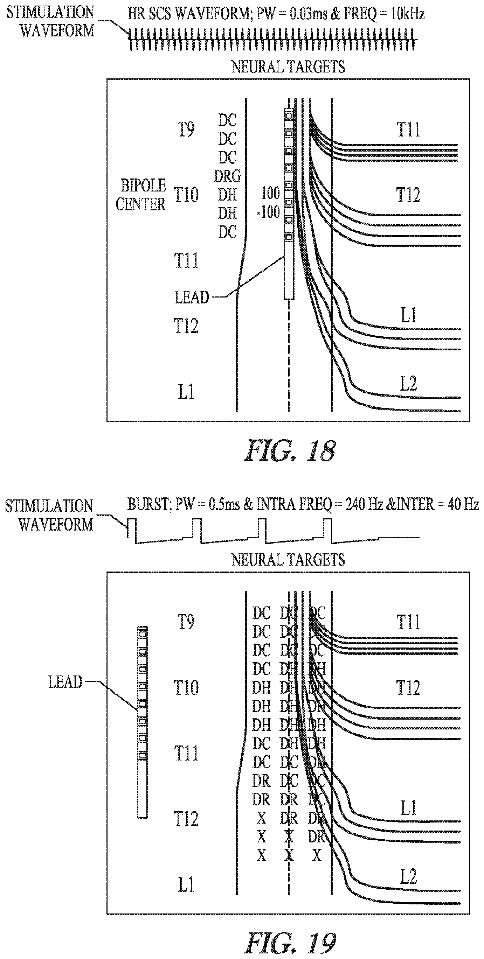

FIGS. 18-24 illustrate various examples of operations of modulation control circuitry 1550 when external programming device 1413 is used by users having various levels of expertise and purposes. In various embodiments, modulation control circuitry 1550 can operate under an operation mode selected from a plurality of operation modes, such as a manual programming mode, a quick programming mode, and a customization programming mode. The operation mode can be directly selected by the user using user input device 1460 and/or automatically selected by modulation control circuitry 1550 when a function related to the operation mode is used by the user.

The manual programming mode may be intended for use by a user who has expert-level knowledge and understanding of the disease and treatment mechanisms related to the use of system 210, including its various embodiments as discussed in this document, as well as the operation of the system. Simulations, academic literature, clinical observations of symptoms may lead to the development of spatial targeting maps that can be used in determining the stimulation configuration. Under the manual programming mode, relationship definition circuitry 1562 can present relationship on presentation device 1459 information known from the stored one or more neuromodulation relationships, and obtain additional relationship information for adjusting the neuromodulation relationship selected from the stored one or more neuromodulation relationships or adding the new neuromodulation relationship to the stored one or more neuromodulation relationships. In various embodiments, relationship definition circuitry 1562 can adjust or add the neuromodulation relationship by performing a simulation using the obtained additional relationship information and one or more computation models selected from the stored computational models. Examples of the known relationship information may include electrode configuration (e.g., when the lead is already implanted in the patient) and target information (specified by the user). Examples of the additional relationship information may include changes to values of parameters in the adjusted neuromodulation relationship, or values of parameters in the added neuromodulation relationship. Such relationship information relates to the waveform parameters, electrode parameters (e.g., electrode selection, fractionalization), and/or target parameters (e.g., dermatomal level, neuro fibers or other targeted neural elements).

FIG. 18 illustrates an example of a display on a user interface, such as user interface 1514, operating under a manual programming mode. For a given lead placement/user target selection, the first target activated by a given lead placement is paired to each possible bipole center. The user is shown partial or full spinal anatomy and the fractionalization. The user selects an electrode design and a dermatomal level with or without the anatomy shown. Mapping of neural target to the bipole or other target pole placements is shown to the user, and the user can then adjust the lead placement using the user interface (e.g., by dragging, point-and-clicking, or double clicking). In FIG. 18, "DC" refers to dorsal column, and "DH" refers to dorsal horn. They designate neural element that will be affected by the lowest amplitude of stimulation (i.e., the most sensitive neural element) given the electrode configuration (in this particular case, the specific element at a location that will be activated by the current electrode placement and configuration) as amplitude is turned up from 0.

FIG. 19 illustrates another example of a display on a user interface, such as user interface 1514, operating under the manual programming mode. The user can specify single or multiple neural fibers, and the display is adjusted, as illustrated for example, the largest number of activated structures in selected groups at a given lead site. Impossible activation (i.e., activation using a given placement and configuration that requires an amplitude, pulse width, and/or other parameters beyond the capabilities of the stimulator) is also shown (by "x").

The quick programming mode may be intended for use by a user performing a routine task of adjusting the programming of a neuromodulation device. Under the quick programming mode, stimulation definition circuitry 1563 can present stimulation information known from the stored patient information and obtain additional stimulation information known to the user. Target definition circuitry 1564 can obtain the target information including disease information (such as pain dermatome and pain etiology) and produce the target parameters using the disease information and a disease-target relationship selected from the one or more disease-target relationships stored in external storage device 1418. For example, target definition circuitry 1564 can present a dermatomal map including selectable dermatomes and to receive a dermatome selected from the selectable dermatomes by the user. Target definition circuitry 1564 can also receive a pain etiology from the user, such as by presenting a list of pain etiologies and receiving the pain etiology selected from the list of pain etiologies by the user. Modulation control circuitry 1550 can present a disease mechanism when desirable, such as when the user desires such knowledge to assist the device programming. Stimulation configuration generation circuitry 1566 can generate and present the stimulation configuration as a recommendation for the stimulation configuration;

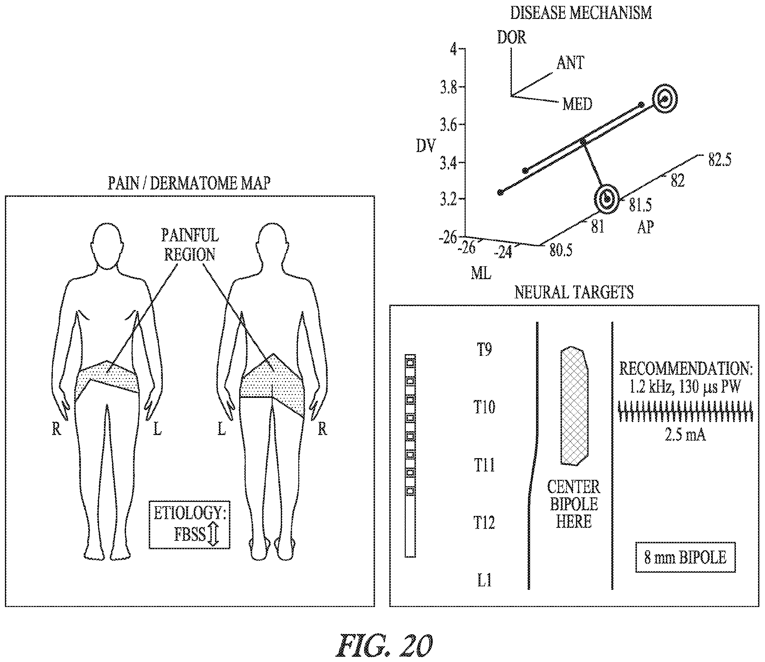

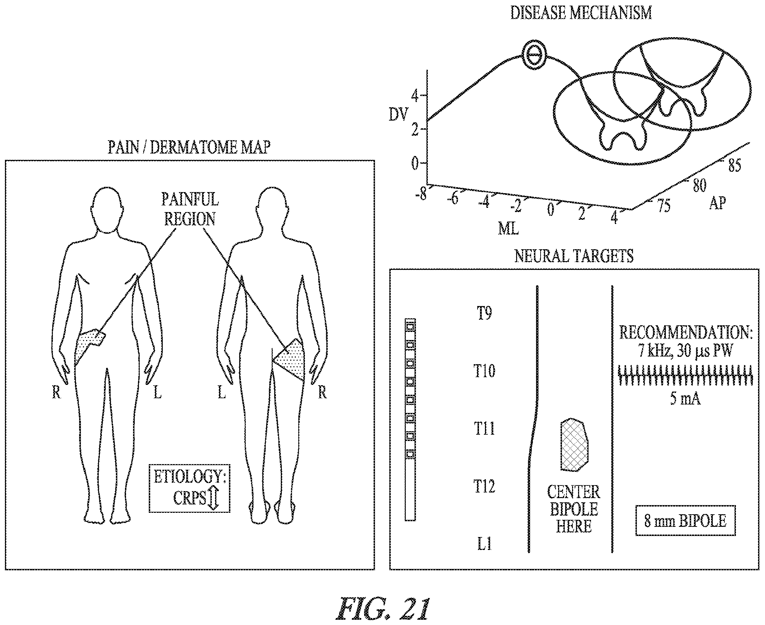

FIG. 20 illustrates an example of a display on a user interface, such as user interface 1514, operating under a quick programming mode. The illustrated example relates to treatment of failed back surgery syndrome (FBSS) by targeted stimulation of dorsal horn elements involved in the disease state. FIG. 21 illustrates another example of a display on a user interface, such as user interface 1514, operating under the quick programming mode. The illustrated example relates to treatment of complex regional pain syndrome (CRPS) by dorsal root activation. In the illustrated examples, the user selects a dermatome and a pain etiology (FBSS and CRPS shown as examples). Neural elements believed to be associated with the pain state mat be shown, and may be selectable in some examples. In the illustrated examples, dermatome maps are shown to allow the user or the patient to identify the painful region by highlighting. Neural target information shown to the user include recommended lead placement, recommended stimulation waveform produced based on back-calculation from the selected neural target (pain dermatomes highlighted by the user or the patient). The recommendation is to be updated as necessary when the user changes any input information.

The customization programming mode may be intended for use by any user who wishes to customize the stimulation configuration for an individual patient. Under the customization mode, customization circuity 1565 can obtain customization information and customize one or more neuromodulation relationships selected from the stored one or more neuromodulation relationships using the customization information. Under the customization mode, the user can also modify the recommended stimulation configuration. Stimulation configuration generation circuitry 1566 can present the generated stimulation configuration as a recommendation for the stimulation configuration, present the target parameters corresponding to the stimulation configuration, and allow the user to adjust the generated stimulation configuration. The user can adjust the stimulation configuration by entering additional stimulation information and/or additional target information for generating a new stimulation configuration, and/or by modifying the stimulation configuration directly, until the user accepts the stimulation configuration. For this purpose, stimulation definition circuitry 1562 can obtain the additional stimulation information and adjust the waveform parameters and/or the electrode parameters using the obtained additional stimulation information. Target definition circuitry 1563 can obtain the additional target information and adjust the target parameters using the obtained additional target information. Stimulation configuration generation circuitry 1566 can generate a modified stimulation configuration based on the adjusted waveform parameters, electrode parameters, and/or target parameters, and a neuromodulation relationship selected from customized one or more neuromodulation relationships. Modulation control circuitry 1550 can present a disease mechanism when desirable, such as when the user desires such knowledge to assist the customization.

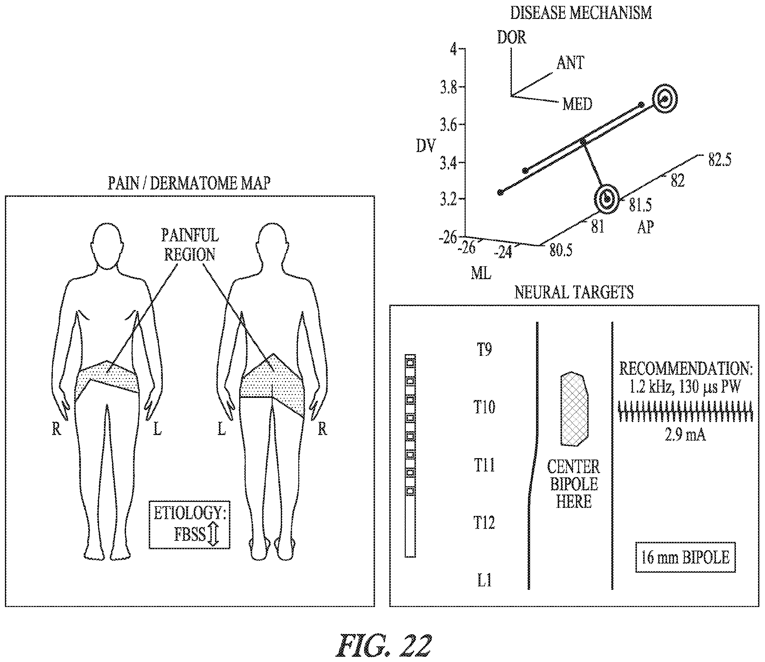

FIG. 22 illustrates an example of a display on a user interface, such as user interface 1514, operating under a customization programming mode. The user can move the target pole. As a result, the stimulation waveform, center bipole location, and minimum amplitude recommendations may change.

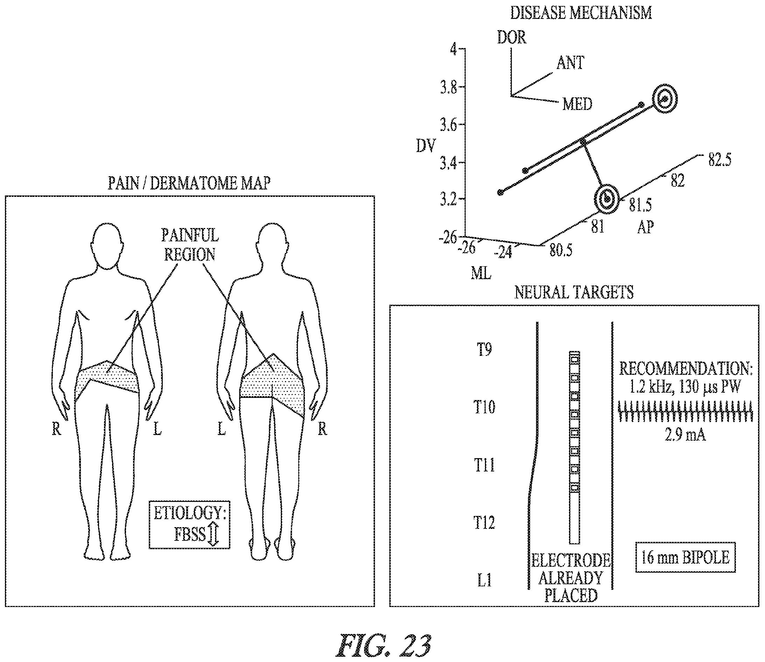

FIG. 23 illustrates another example of a display on a user interface, such as user interface 1514, operating under the customization programming mode. If a painful region has been defined, the lead has been already placed, and a stimulation waveform is recommended, a neuromodulation relationship (such as the LUT shown in FIG. 17) can be used to produce a recommended stimulation configuration including a recommended stimulation geometry with a recommended fractionalization based on disease state.

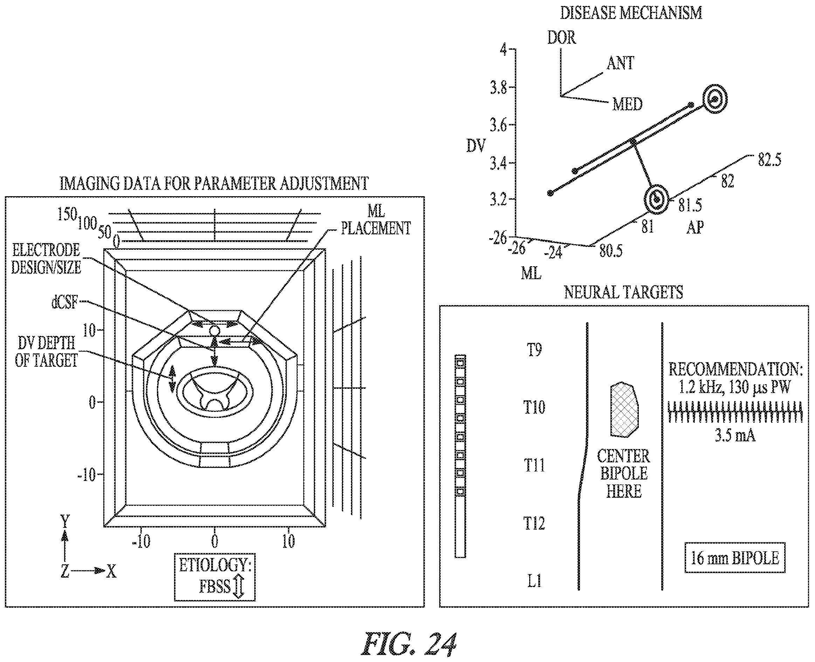

FIG. 24 illustrates an example of a display on a user interface, such as user interface 1514, operating under the customization programming mode. In the illustrated example, patient-specific information such as imaging data is used for customization of the stimulation configuration. Such patient-specific information may include parameters each representing a key input into a finite element model/neuron model pair from which lead placement and stimulation waveform recommendations are simulated and derived. Change in dorsal CSF ("dSCF") depth (e.g., from 3.2 mm to 4.2 mm) results in increase in the recommended amplitude of the stimulation waveform and change in the recommended center bipole location. Specific changes are based on outputs of offline finite element modeling and/or online electrostatic calculations.

In various embodiments, circuits of system 210, including its various embodiments discussed in this document, may be implemented using a combination of hardware and software. For example, the circuits may be implemented using an application-specific circuit constructed to perform one or more particular functions or a general-purpose circuit programmed to perform such function(s). Such a general-purpose circuit includes, but is not limited to, a microprocessor or a portion thereof, a microcontroller or portions thereof, and a programmable logic circuit or a portion thereof.

It is to be understood that the above detailed description is intended to be illustrative, and not restrictive. Other embodiments will be apparent to those of skill in the art upon reading and understanding the above description. The scope of the invention should, therefore, be determined with reference to the appended claims.

## Claims

1. A system (210; 520; 624) for delivering neurostimulation to tissue of a patient using a stimulation device (212; 312; 512; 626; 1312) and a plurality of electrodes (211; 311; 511; 631; 731; 1044; E1-E16) and controlling the delivery of the neurostimulation by a user, the system comprising:
an external programming device (213; 413; 1413), the external programming device (213; 413; 1413) comprising:
a programming control circuit (419; 1419) configured to program the stimulation device for delivering the neurostimulation through one or more electrodes of the plurality of electrodes according to a stimulation configuration specified by a waveform parameter set defining a waveform of the neurostimulation and an electrode parameter set defining an electrode configuration of the neurostimulation;
an external storage device (418; 1418) configured to store one or more neuromodulation relationships and one or more computational models of one or more neural targets, the one or more neuromodulation relationships each relating one or more candidate stimulation configurations to the one or more neural targets, the one or more candidate stimulation configurations each selectable to be the stimulation configuration for programming the stimulation device, the one or more neural targets each specified by a target parameter set; and
a user interface (414; 1414; 1514) coupled to the programming control circuit and the external storage device, the user interface including modulation control circuitry (450; 1550) configured to:
obtain relationship information;
add or adjust a neuromodulation relationship of the stored one or more neuromodulation relationships by performing a simulation using the obtained relationship information and one or more computational models selected from the stored one or more computational models of the one or more neural targets;
obtain two parameter sets selected from a group consisting of the waveform parameter set, the electrode parameter set, and the target parameter set; and determine the other parameter set selected from the group consisting of the waveform parameter set, the electrode parameter set, and the target parameter set using the two obtained parameter sets and a neuromodulation relationship selected from the stored one or more neuromodulation relationships.

2. The system according to claim 1, wherein the modulation control circuitry (450; 1550) is configured to obtain the waveform parameter set and the electrode parameter set, and to determine the target parameter set using the waveform parameter set, the electrode parameter set, and the selected neuromodulation relationship.

3. The system according to claim 1, wherein the modulation control circuitry (450; 1550) is configured to obtain the waveform parameter set and the target parameter set, and to determine the electrode parameter set using the waveform parameter set, the target parameter set, and the selected neuromodulation relationship.

4. The system according to claim 1, wherein the modulation control circuitry (450; 1550) is configured to obtain the target parameter set and the electrode parameter set, and to determine the waveform parameter set using the target parameter set, the electrode parameter set, and the selected neuromodulation relationship.

5. The system according to any of the preceding claims, wherein the modulation control circuitry (450; 1550) further comprises:
stimulation definition circuitry (1563) configured to obtain stimulation information and to produce one or more values for one or more parameters of the waveform parameter set and the electrode parameter set using the obtained stimulation information; and
target definition circuitry (1564) configured to obtain target information and produce one or more values for one or more parameters of the target parameter set using the obtained target information.

6. The system according to claim 5, wherein the modulation control circuitry (450; 1550) further comprises stimulation configuration generation circuitry (1566) configured to generate the stimulation configuration for programming the stimulation device based on the produced one or more values for one or more parameters of the waveform parameter set, the electrode parameter set, and the target parameter set and a neuromodulation relationship selected from the stored one or more neuromodulation relationships.

7. The system according to claim 6, wherein the user interface (414; 1414; 1514) comprises a user input device (1460), and the stimulation configuration generation circuitry (1566) is further configured to allow the user to select the neuromodulation relationship using the user input device.

8. The system according to claim 6, wherein the stimulation configuration generation circuitry (1566) is further configured to select the neuromodulation relationship automatically based on one or more of the obtained stimulation information or the obtained target information.

9. The system according to any of claims 6 to 8, wherein the user interface (414; 1414; 1514) comprises a presentation device (/1459), and the stimulation configuration generation circuitry (1566) is further configured to present on the presentation device the generated stimulation configuration as a recommendation for the stimulation configuration for programming the stimulation device.

10. The system according to claim 9, wherein the stimulation configuration generation circuitry (1566) is further configured to present a representation of the target parameter set corresponding to the recommended stimulation configuration.

11. The system according to claim 9 or 10, wherein the stimulation configuration generation circuitry (1566) is further configured to allow the user to adjust the generated stimulation configuration.

12. The system according to any of claims 5 to 11, wherein the external storage
device (418; 1418) is further configured to store one or more disease-target relationships relating disease information indicative of one or more neural disorders to the neural targets, and the target definition circuitry (1564) is further configured to obtain the disease information as the target information and to produce the target parameters using the disease information and a disease-target relationship selected from the stored one or more disease-target relationships.

13. The system according to any of the preceding claims, wherein the modulation control circuitry (450; 1550) further comprises customization circuity (1565) configured to obtain customization information and customize one or more neuromodulation relationship selected from the stored one or more neuromodulation relationships using the customization information.

## Patentansprüche

1. System (210; 520; 624) zur Abgabe einer Neurostimulation an Gewebe eines Patienten unter Verwendung einer Stimulationsvorrichtung (212; 312; 512; 626; 1312) und mehrerer Elektroden (211; 311; 511; 631; 731; 1044; E1-E16) und zum Steuern der Abgabe der Neurostimulation durch einen Benutzer, wobei das System aufweist:
eine externe Programmiervorrichtung (213; 413; 1413), wobei die externe Programmiervorrichtung (213; 413; 1413) aufweist:
eine Programmiersteuerschaltung (419; 1419), die eingerichtet ist, um die Stimulationsvorrichtung zur Abgabe der Neurostimulation über eine oder mehrere Elektroden der mehreren Elektroden gemäß einer Stimulationskonfiguration zu programmieren, die durch einen Wellenform-Parametersatz, der eine Wellenform der Neurostimulation definiert, und einen Elektroden-Parametersatz, der eine Elektrodenkonfiguration der Neurostimulation definiert, angegeben ist;
eine externe Speichervorrichtung (418; 1418), die eingerichtet ist, um eine oder mehrere Neuromodulationsbeziehungen und ein oder mehrere Berechnungsmodelle von einem oder mehreren neuronalen Zielen zu speichern, wobei die eine oder mehreren Neuromodulationsbeziehungen jeweils eine oder mehrere Kandidatenstimulationskonfigurationen mit dem einen oder den mehreren neuronalen Zielen in Beziehung setzen, wobei die eine oder mehreren Kandidatenstimulationskonfigurationen jeweils als die Stimulationskonfiguration zur Programmierung der Stimulationsvorrichtung auswählbar sind, wobei das eine oder die mehreren neuronalen Ziele jeweils durch einen Ziel-Parametersatz angegeben sind; und
eine Benutzerschnittstelle (414; 1414; 1514), die mit der Programmiersteuerschaltung und der externen Steuerschaltung gekoppelt ist, wobei die Benutzerschnittstelle eine Modulationssteuerschaltungsanordnung (450; 1550) aufweist, die eingerichtet ist zum:
Erhalten von Beziehungsinformationen;
Hinzufügen oder Einstellen einer Neuromodulationsbeziehung der einen oder mehreren gespeicherten Neuromodulationsbeziehungen, indem eine Simulation unter Verwendung der erhaltenen Beziehungsinformationen und eines oder mehrerer Berechnungsmodelle, die aus dem einen oder den mehreren gespeicherten Berechnungsmodellen des einen oder der mehreren neuronalen Ziele ausgewählt werden, durchgeführt wird;
Erhalten von zwei Parametersätzen, die aus einer Gruppe ausgewählt werden, die aus dem Wellenform-Parametersatz, dem Elektroden-Parametersatz und dem Ziel-Parametersatz besteht; und
Bestimmen des anderen Parametersatzes, der aus der Gruppe ausgewählt wird, die aus dem Wellenform-Parametersatz, dem Elektroden-Parametersatz und dem Ziel-Parametersatz besteht, unter Verwendung der zwei erhaltenen Parametersätze und einer Neuromodulationsbeziehung, die aus der einen oder den mehreren gespeicherten Neuromodulationsbeziehungen ausgewählt wird.

2. System nach Anspruch 1, wobei die Modulationssteuerschaltungsanordnung (450; 1550) eingerichtet ist, um den Wellenform-Parametersatz und den Elektroden-Parametersatz zu erhalten und um den Ziel-Parametersatz unter Verwendung des Wellenform-Parametersatzes, des Elektroden-Parametersatzes und der ausgewählten Neuromodulationsbeziehung zu bestimmen.

3. System nach Anspruch 1, wobei die Modulationssteuerschaltungsanordnung (450; 1550) eingerichtet ist, um den Wellenform-Parametersatz und den Ziel-Parametersatz zu erhalten und um den Elektroden-Parametersatz unter Verwendung des Wellenform-Parametersatzes, des Ziel-Parametersatzes und der ausgewählten Neuromodulationsbeziehung zu bestimmen.

4. System nach Anspruch 1, wobei die Modulationssteuerschaltungsanordnung (450; 1550) eingerichtet ist, um den Ziel-Parametersatz und den Elektroden-Parametersatz zu erhalten und um den Wellenform-Parametersatz unter Verwendung des Ziel-Parametersatzes, des Elektroden-Parametersatzes und der ausgewählten Neuromodulationsbeziehung zu bestimmen.

5. System nach einem der vorhergehenden Ansprüche, wobei die Modulationssteuerschaltungsanordnung (450; 1550) ferner aufweist:
eine eine Stimulation definierende Schaltungsanordnung (1563), die eingerichtet ist, um Stimulationsinformationen zu erhalten und um einen oder mehrere Werte für einen oder mehrere Parameter des Wellenform-Parametersatzes und des Elektroden-Parametersatzes unter Verwendung der erhaltenen Stimulationsinformationen zu erzeugen; und
eine ein Ziel definierende Schaltungsanordnung (1564), die eingerichtet ist, um Zielinformationen zu erhalten und um einen oder mehrere Werte für einen oder mehrere Parameter des Ziel-Parametersatzes unter Verwendung der erhaltenen Zielinformationen zu erzeugen.

6. System nach Anspruch 5, wobei die Modulationssteuerschaltungsanordnung (450; 1550) ferner eine eine Stimulationskonfiguration erzeugende Schaltungsanordnung (1566) umfasst, die eingerichtet ist, um die Stimulationskonfiguration zur Programmierung der Stimulationsvorrichtung basierend auf dem einen oder den mehreren erzeugten Werten für einen oder mehrere Parameter des Wellenform-Parametersatzes, des Elektroden-Parametersatzes und des Ziel-Parametersatzes und einer Neuromodulationsbeziehung, die aus der einen oder den mehreren gespeicherten Neuromodulationsbeziehungen ausgewählt wird, zu erzeugen.

7. System nach Anspruch 6, wobei die Benutzerschnittstelle (414; 1414; 1514) eine Benutzereingabevorrichtung (1460) aufweist und die eine Stimulationskonfiguration erzeugende Schaltungsanordnung (1566) ferner eingerichtet ist, um zu ermöglichen, dass der Benutzer die Neuromodulationsbeziehung unter Verwendung der Benutzereingabevorrichtung auswählt.

8. System nach Anspruch 6, wobei die eine Stimulationskonfiguration erzeugende Schaltungsanordnung (1566) ferner eingerichtet ist, um die Neuromodulationsbeziehung automatisch basierend auf einer oder mehreren der erhaltenen Stimulationsinformationen oder der erhaltenen Zielinformationen auszuwählen.

9. System nach einem der Ansprüche 6 bis 8, wobei die Benutzerschnittstelle (414; 1414; 1514) eine Präsentationsvorrichtung (1459) aufweist und die eine Stimulationskonfiguration erzeugende Schaltungsanordnung (1566) ferner eingerichtet ist, um auf der Präsentationsvorrichtung die erzeugte Stimulationskonfiguration als eine Empfehlung für die Stimulationskonfiguration zur Programmierung der Stimulationsvorrichtung zu präsentieren.

10. System nach Anspruch 9, wobei die eine Stimulationskonfiguration erzeugende Schaltungsanordnung (1566) ferner eingerichtet ist, um eine Darstellung des Ziel-Parametersatzes zu präsentieren, welcher der empfohlenen Stimulationskonfiguration entspricht.

11. System nach Anspruch 9 oder 10, wobei die eine Stimulationskonfiguration erzeugende Schaltungsanordnung (1566) ferner eingerichtet ist, um zu ermöglichen, dass der Benutzer die erzeugte Stimulationskonfiguration einstellt.

12. System nach einem der Ansprüche 5 bis 11, wobei die externe Speichervorrichtung (418; 1418) ferner eingerichtet ist, um eine oder mehrere Krankheit-Ziel-Beziehungen zu speichern, welche Krankheitsinformationen, die eine oder mehrere neuronale Störungen angeben, mit den neuronalen Zielen in Beziehung setzen, und wobei die ein Ziel definierende Schaltungsanordnung (1564) ferner eingerichtet ist, um die Krankheitsinformationen als die Zielinformationen zu erhalten und um die Zielparameter unter Verwendung der Krankheitsinformationen und einer Krankheit-Ziel-Beziehung, die aus der einen oder den mehreren gespeicherten Krankheit-Ziel-Beziehungen ausgewählt wird, zu erzeugen.

13. System nach einem der vorhergehenden Ansprüche, wobei die Modulationssteuerschaltungsanordnung (450; 1550) ferner eine Anpassungsschaltungsanordnung (1565) aufweist, die eingerichtet ist, um Anpassungsinformationen zu erhalten und um eine oder mehrere Neuromodulationsbeziehungen, die aus der einen oder den mehreren gespeicherten Neuromodulationsbeziehungen ausgewählt werden, unter Verwendung der Anpassungsinformationen anzupassen.

## Revendications

1. Système (210 ; 520 ; 624) destiné à délivrer une neurostimulation à un tissu d'un patient, en utilisant un dispositif de stimulation (212 ; 312 ; 512 ; 626 ; 1312) et une pluralité d'électrodes (211 ; 311 ; 511 ; 631 ; 731 ; 1044 ; E1-E16), et à commander la délivrance de la neurostimulation par le biais d'un utilisateur, le système comprenant :
un dispositif de programmation externe (213 ; 413 ; 1413), le dispositif de programmation externe (213 ; 413 ; 1413) comprenant :
un circuit de commande de programmation (419 ; 1419) configuré de manière à programmer le dispositif de stimulation pour délivrer la neurostimulation à travers une ou plusieurs électrodes de la pluralité d'électrodes selon une configuration de stimulation spécifiée par un ensemble de paramètres de forme d'onde définissant une forme d'onde de la neurostimulation et un ensemble de paramètres d'électrode définissant une configuration d'électrode de la neurostimulation ;
un dispositif de stockage externe (418 ; 1418) configuré de manière à stocker une ou plusieurs relations de neuromodulation et un ou plusieurs modèles informatiques d'une ou plusieurs cibles neuronales, ladite une ou lesdites plusieurs relations de neuromodulation reliant chacune une ou plusieurs configurations de stimulation candidates à ladite une ou auxdites plusieurs cibles neuronales, ladite une ou lesdites plusieurs configurations de stimulation candidates pouvant chacune être sélectionnées pour être la configuration de stimulation pour la programmation du dispositif de stimulation, ladite une ou lesdites plusieurs cibles neuronales étant chacune spécifiées par un ensemble de paramètres de cible ; et
une interface utilisateur (414 ; 1414 ; 1514) couplée au circuit de commande de programmation et au dispositif de stockage externe, l'interface utilisateur incluant un montage de circuits de commande de modulation (450 ; 1550) configuré de manière à :
obtenir des informations de relation ;
ajouter ou ajuster une relation de neuromodulation parmi ladite une ou lesdites plusieurs relations de neuromodulation stockées, en mettant en œuvre une simulation au moyen des informations de relation obtenues et d'un ou plusieurs modèles informatiques sélectionnés parmi ledit un ou lesdits plusieurs modèles informatiques stockés de ladite une ou desdites plusieurs cibles neuronales ;
obtenir deux ensembles de paramètres sélectionnés à partir d'un groupe constitué de l'ensemble de paramètres de forme d'onde, de l'ensemble de paramètres d'électrode et de l'ensemble de paramètres de cible ; et
déterminer l'autre ensemble de paramètres sélectionné à partir du groupe constitué de l'ensemble de paramètres de forme d'onde, de l'ensemble de paramètres d'électrode et de l'ensemble de paramètres de cible, en utilisant les deux ensembles de paramètres obtenus et une relation de neuromodulation sélectionnée parmi ladite une ou lesdites plusieurs relations de neuromodulation stockées.

2. Système selon la revendication 1, dans lequel le montage de circuits de commande de modulation (450 ; 1550) est configuré de manière à obtenir l'ensemble de paramètres de forme d'onde et l'ensemble de paramètres d'électrode, et à déterminer l'ensemble de paramètres de cible en utilisant l'ensemble de paramètres de forme d'onde, l'ensemble de paramètres d'électrode, et la relation de neuromodulation sélectionnée.

3. Système selon la revendication 1, dans lequel le montage de circuits de commande de modulation (450 ; 1550) est configuré de manière à obtenir l'ensemble de paramètres de forme d'onde et l'ensemble de paramètres de cible, et à déterminer l'ensemble de paramètres d'électrode en utilisant l'ensemble de paramètres de forme d'onde, l'ensemble de paramètres de cible et la relation de neuromodulation sélectionnée.

4. Système selon la revendication 1, dans lequel le montage de circuits de commande de modulation (450 ; 1550) est configuré de manière à obtenir l'ensemble de paramètres de cible et l'ensemble de paramètres d'électrode, et à déterminer l'ensemble de paramètres de forme d'onde en utilisant l'ensemble de paramètres de cible, l'ensemble de paramètres d'électrode et la relation de neuromodulation sélectionnée.

5. Système selon l'une quelconque des revendications précédentes, dans lequel le montage de circuits de commande de modulation (450 ; 1550) comprend en outre :
un montage de circuits de définition de stimulation (1563) configuré de manière à obtenir des informations de stimulation et à produire une ou plusieurs valeurs pour un ou plusieurs paramètres de l'ensemble de paramètres de forme d'onde et de l'ensemble de paramètres d'électrode, en utilisant les informations de stimulation obtenues ; et
un montage de circuits de définition de cible (1564) configuré de manière à obtenir des informations de cible et à produire une ou plusieurs valeurs pour un ou plusieurs paramètres de l'ensemble de paramètres de cible, en utilisant les informations de cible obtenues.

6. Système selon la revendication 5, dans lequel le montage de circuits de commande de modulation (450 ; 1550) comprend en outre un montage de circuits de génération de configuration de stimulation (1566) configuré de manière à générer la configuration de stimulation pour programmer le dispositif de stimulation sur la base de ladite une ou desdites plusieurs valeurs produites pour un ou plusieurs paramètres de l'ensemble de paramètres de forme d'onde, de l'ensemble de paramètres d'électrode, et de l'ensemble de paramètres de cible, et d'une relation de neuromodulation sélectionnée à partir de ladite une ou desdites plusieurs relations de neuromodulation stockées.

7. Système selon la revendication 6, dans lequel l'interface utilisateur (414 ; 1414 ; 1514) comprend un dispositif d'entrée utilisateur (1460), et le montage de circuits de génération de configuration de stimulation (1566) est en outre configuré de manière à permettre à l'utilisateur de sélectionner la relation de neuromodulation en utilisant le dispositif d'entrée utilisateur.

8. Système selon la revendication 6, dans lequel le montage de circuits de génération de configuration de stimulation (1566) est en outre configuré de manière à sélectionner la relation de neuromodulation automatiquement, sur la base d'une ou plusieurs informations parmi les informations de stimulation obtenues et les informations de cible obtenues.

9. Système selon l'une quelconque des revendications 6 à 8, dans lequel l'interface utilisateur (414 ; 1414 ; 1514) comprend un dispositif de présentation (1459), et le montage de circuits de génération de configuration de stimulation (1566) est en outre configuré de manière à présenter, sur le dispositif de présentation, la configuration de stimulation générée, sous la forme d'une recommandation concernant la configuration de stimulation permettant de programmer le dispositif de stimulation.

10. Système selon la revendication 9, dans lequel le montage de circuits de génération de configuration de stimulation (1566) est en outre configuré de manière à présenter une représentation de l'ensemble de paramètres de cible correspondant à la configuration de stimulation recommandée.

11. Système selon la revendication 9 ou 10, dans lequel le montage de circuits de génération de configuration de stimulation (1566) est en outre configuré de manière à permettre à l'utilisateur d'ajuster la configuration de stimulation générée.

12. Système selon l'une quelconque des revendications 5 à 11, dans lequel le dispositif de stockage externe (418 ; 1418) est en outre configuré de manière à stocker une ou plusieurs relations « maladie-cible » mettant en relation des informations de maladie, indicatives d'un ou plusieurs troubles neuronaux, avec les cibles neuronales, et dans lequel le montage de circuits de définition de cible (1564) est en outre configuré de manière à obtenir les informations de maladie, sous la forme des informations de cible, et à produire les paramètres de cible en utilisant les informations de maladie et une relation « maladie-cible » sélectionnée parmi ladite une ou lesdites plusieurs relations « maladie-cible » stockées.

13. Système selon l'une quelconque des revendications précédentes, dans lequel le montage de circuits de commande de modulation (450 ; 1550) comprend en outre un montage de circuits de personnalisation (1565) configuré de manière à obtenir des informations de personnalisation et à personnaliser une ou plusieurs relations de neuromodulation sélectionnées parmi ladite une ou lesdites plusieurs relations de neuromodulation stockées, en utilisant les informations de personnalisation.
